# EUROPÄISCHE PATENTSCHRIFT

(11) **EP 2 101 175 B1**
(45) Veröffentlichungstag und Bekanntmachung des Hinweises auf die Patenterteilung: **16.05.2012**
(21) Anmeldenummer: 09100171.9
(22) Anmeldetag: 10.03.2009
(51) Int. Cl.: G01N 33/543, G01N 33/542, G01N 33/58, C07K 14/32

(54) **Hochgeordnete Nanostruktur und Sensor und deren Verwendung**
High order nanostructure and sensor and use of same
Nanostructure à ordre élevé et capteur ainsi que leur utilisation

(30) Priorität: 10.03.2008 DE 102008014298
(43) Veröffentlichungstag der Anmeldung: 16.09.2009
(73) Patentinhaber: Helmholtz-Zentrum Dresden - Rossendorf e.V., 01328 Dresden (DE)
(72) Erfinder: Pollmann, Dr. Katrin, 01324, Dresden (DE); Fahmy, Dr. Karim, 01324, Dresden (DE); Raff, Dr. Johannes, 01324, Dresden (DE)
(74) Vertreter: Kailuweit & Uhlemann Patentanwälte

(56) Entgegenhaltungen:
- WO-A-02/061392
- WO-A-02/097118
- PLESCHBERGER MAGDALENA ET AL: "An S-layer heavy chain camel antibody fusion protein for generation of a nanopatterned sensing layer to detect the prostate-specific antigen by surface plasmon resonance technology." BIOCONJUGATE CHEMISTRY 2004 MAY-JUN, Bd. 15, Nr. 3, Mai 2004 (2004-05), Seiten 664-671, XP002529464 ISSN: 1043-1802
- SLEYTR UWE B ET AL: "S-layers as a tool kit for nanobiotechnological applications." FEMS MICROBIOLOGY LETTERS FEB 2007, Bd. 267, Nr. 2, Februar 2007 (2007-02), Seiten 131-144, XP002529465 ISSN: 0378-1097
- POLLMANN, K. ET AL.: "Bacterial surface layers (S-layers) as building blocks for nanocomposites" INTERNET ARTICLE, [Online] 18. September 2007 (2007-09-18), XP002529466 Gefunden im Internet: URL:http://www.fzd.de/pls/rois/!Publicatio ns?pSelAuthor=%22Grosse%2C%20E.%22&pLang=e n&pSelSort=TITEL&pNid=157&pSelWithSubmitte d=0&pSelPage=10> [gefunden am 2009-05-26]

## Beschreibung

Die vorliegende Erfindung betrifft eine hochgeordnete Struktur und einen Sensor, in der bzw. dem organische Molekülgruppen, insbesondere Liganden (wie z. B. Aptamere), die spezifische Bindungseigenschaften für ein Zielmolekül haben, geordnet gebunden sind, und deren Verwendung.

Anwendungsgebiete der Erfindung finden sich insbesondere auf den Gebieten der Biotechnologie, der Verfahrenstechnik, der Umwelttechnik und der Pharmazie. Die erfindungsgemäße Struktur eignet sich insbesondere zur Herstellung von Biosensoren, für die biomedizinische Analytik, zur Beseitigung von Schadstoffen und Pathogenen, für die DNA-Analytik, für die Herstellung von Filtermaterialien, für Separationsverfahren oder für Katalysatoren, und in der medizinischen Therapie für den gezielten Transport eines Wirkstoffs an seinen Zielort (sog. "drug targeting").

Organische Molekülgruppen, die spezifische Bindungseigenschaften für definierte Zielmoleküle haben, sind aus dem Stand der Technik bekannt. Neben Antikörpern sind dies insbesondere sogenannte Aptamere, d. h. einzelsträngige Oligonukleotide im Größenbereich zwischen 25 und 70 Nukleotiden, die z. B. durch *in vitro-*Selektionsverfahren (auch als SELEX-Verfahren bekannt) identifiziert werden und über ihre dreidimensionale Struktur sehr spezifisch an unterschiedlichste chemische Verbindungen binden.

WO02/097118 A1 offenbart ein Verfahren zur Beschichtung von Trägermaterialien mit S-Layer-Proteinen durch elektrochemische Abscheidung. Die S-Layer-Proteinschicht kann als Träger für funktionelle Moleküle verwendet werden. Die funktionellen Moleküle werden dabei entweder vor der Abscheidung auf ein Substrat an die S-Layer-Proteine gebunden oder nach der Ausbildung einer kristallinen Struktur. Die funktionellen Moleküle werden dabei an Positionen abgelegt, die durch die kristalline Struktur vorgegeben sind.

WO 02/061392 A2 offenbart ein Verfahren und eine Vorrichtung zur Detektion von Analyten in Flüssigkeiten. Auf einem Substrat wird ein Sensorarray mit Kavitäten durch Ätztechnolgie hergestellt. In diese werden chemisch empfindliche Partikel positioniert, die in einer Ausführungsform mit organischen Molekülen als Rezeptoren kombiniert werden, und bei Interaktion mit dem Analyt ein optisches Signal abgegeben.

Bisher verwendete Verfahren zur Herstellung von Sensorschichten bedienen sich einzelner Moleküle, die über mehrfache Modifikation funktionalisiert werden müssen. Neben dem Einführen einer Funktionalität zur Bindung des Zielmoleküls und dem Einführen einer weiteren Funktionalität zur Detektion (z. B. einem Enzym oder einem Fluoreszenzfarbstoff) sind weitere Verfahrensschritte notwendig, um die Moleküle auf einer Schicht anzuordnen.
Nachteile der bisher bekannten Verfahren sind der hohe Aufwand bei der Herstellung von Oberflächen, die mit regelmäßigen Nanostrukturen belegt sind und an die organische Gruppen angebunden werden können, die Schwierigkeiten bei der sequenziellen Modifikation und der gleichzeitigen Verknüpfung mit regelmäßig angeordneten anorganischen Nanopartikeln. Über diese Verfahren ist die Realisierung regelmäßiger Strukturen verschiedener Periodizität nur bedingt möglich.

Bisher bekannte Anwendungen von S-Layer-Proteinen für nanobiotechnologische Applikationenen werden in einem Übersichtsartikel von Sleytr U. B. et al. 2007 (FEMS Microbiol. Lett 267:131-144) zusammengefasst. Pleschberger M et al. 2004 (Bioconjugate Chem 15:664-671) beschreibt die Anwendung eines Fusionsproteins aus einem S-Layer-Protein und einer Schweren Kette eines Antikörper, der das Prostata-Spezifsche Antigen (PSA) erkennt, in der Oberflächenplasmonenresonanz-Spektroskopie (Surface Plasmon Resonance, SPR) zum Nachweis von PSA.

Die Aufgabe der Erfindung ist es, eine Struktur zur Verfügung zu stellen, in der organische Molekülgruppen, insbesondere Molekülgruppen, die spezifische Bindungseigenschaften haben (wie z. B. Aptamere), geordnet gebunden sind, und dadurch die von den organischen Molekülgruppen gebundenen Substanzen in die Nähe funktioneller Bestandteile zu bringen, mit denen sie in spezitische Wechselwirkung treten.

Erfindungsgemäß wird diese Aufgabe gelöst durch die Merkmale des Hauptanspruchs und die nebengeordneten Ansprüche.

Gegenstand der Erfindung ist eine Nanostruktur gemäß Anspruch 1, enthaltend:
a) S-Layer-Proteine, an die organische Molekülgruppen gebunden sind, die spezifische Bindungseigenschaften für ein Zielmolekül aufweisen und
b) an die S-Layer-Proteine gebundene anorganische Nanopartikel und/oder anorganische bzw. organische Fluoreszenzfarbstoffe oder weitere Biomoleküle, die als funktionelle Bestandteile unterschiedliche Funktionen erfüllen,
   wobei die S-Layer-Proteine eine kristalline Schicht auf einer Oberfläche eines Substrats ausbilden oder in einer geeigneten Flüssigkeit als Suspension vorliegen,
   wobei die Nukleinsäure-Aptamere und/oder Antikörper so geordnet an die S-Layer-Proteine gebunden sind, dass sie die gebundenen Zielmoleküle in die Nähe der funktionellen Bestandteile bringen, die in spezifische Wechselwirkung mit dem Zielmolekül (Analyten) treten.

Die Nanostruktur bildet dabei eine Schicht auf der Oberfläche eines Substrats oder eine Suspension, bevorzugt in einem wässrigen Medium.

Erfindungsgemäß werden organische Molekülgruppen, welche spezifische Bindungseigenschaften für ein Zielmolekül aufweisen, an S-Layer-Proteine gebunden. Dadurch, dass diese S-Layer-Proteine eine geordnete Struktur ausbilden, ergibt sich vorteilhaft eine sehr geordnete Anordnung der organischen Molekülgruppen und die Möglichkeit der sequenziellen Modifizierung der Struktur z. B. durch sequenzielles Anbinden von Liganden.

Die Liganden, d. h. Molekülgruppen, die spezifische Bindungseigenschaften für ein Zielmolekül aufweisen, sind Aptamere und Antikörper, welche es ermöglichen das Zielmolekül selektiv an die Struktur zu binden. Der Begriff Antikörper umfasst dabei auch rekombinant hergestellte Antikörper (wie z. B. scFv - single chain variable Fragment) und andere Antikörper-Fragmente (wie z. B. Fab und F(ab')₂-Fragmente).

Erfindungsgemäß werden an die S-Layer-Proteine weiter Nanopartikel und/oder anorganische bzw. organische Fluoreszenzfarbstoffe oder weitere Biomoleküle (wie z. B. Enzyme) gebunden, die als funktionelle Bestandteile unterschiedliche Funktionen erfüllen, wie z. B. magnetische Abtrennung, enzymatische bzw. katalytische, insbesondere photokatalytische Zersetzung des Zielmoleküls oder Nachweis des Zielmoleküls.

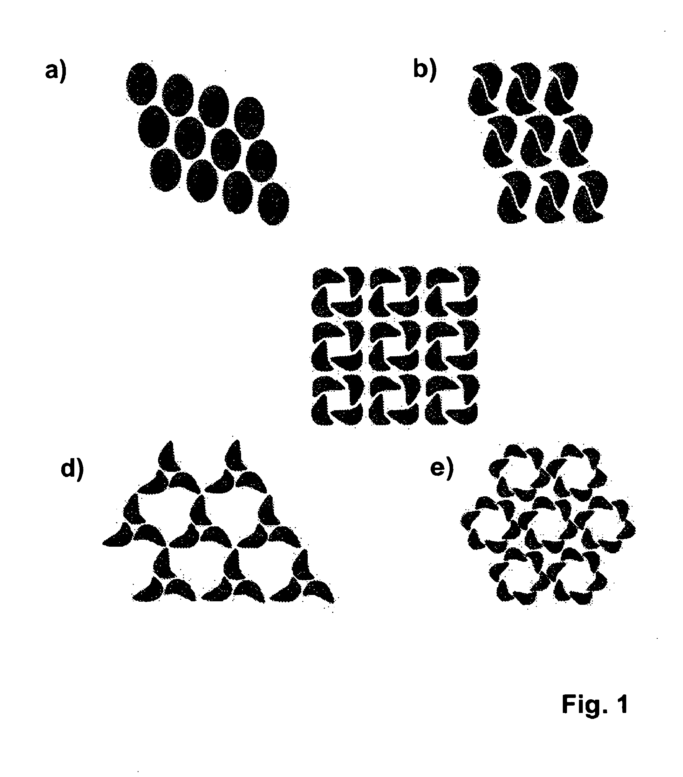

Bevorzugt werden bakterielle S-Layer-Proteine aus den Gattungen *Bacillus, Geobacillus, Lactobacillus, Lysinibacillus, Deinococcus, Sporosarcina* verwendet. Die Besonderheit von S-Layer-Proteinen besteht in ihrer Fähigkeit, sich selber zu hochgeordneten Nanostrukturen, den sog. S-Layern, zu organisieren. Dabei lagern sich die Proteinmonomere zu parakristallinen Gitterstrukturen zusammen, die verschiedene Symmetrien aufweisen können. Dies kann eine schräge (Fig. 1a, b), eine quadratische (Fig. 1c) oder eine hexagonale (Fig. 1d, e) Symmetrie sein. Die Gitterkonstanten betragen 5 nm bis 40 nm und häufig 12 nm bis 20 nm. Die Proteingitter besitzen regelmäßig angeordnete Poren einheitlicher Größe mit einem Durchmesser von 2 nm bis 8 nm. Damit steht mit dem S-Layer eine komplexe selbstorganisierende Nanostruktur zur Verfügung, die mit technischen Mitteln nicht oder nur sehr aufwendig hergestellt werden kann.

Die durch die S-Layer-Proteine gebildete erfindungsgemäße Struktur bildet bevorzugt eine Schicht auf der Oberfläche eines Substrats, bevorzugt Glas, Glimmer, Magnesiumoxid, Aluminiumoxid, Silizium oder Siliziumdioxid. S-Layer-Proteine rekristallisieren außerdem an Grenzflächen zu Monolagen. Sie eignen sich zur Beschichtung von Oberflächen wie Silizium, Glas, Kohlenstofffasern. Durch chemische Modifizierung wie z. B. Aminierung, Silanisierung, Anbindung von sekundären Zellwandpolymeren (SCWPs) oder Beschichtung mit Polymeren wird eine stabile Anbindung an Oberflächen begünstigt und das Materialspektrum an möglichen Substraten erweitert. Die Proteinschicht ist bevorzugt eine Monolayer (d. h. hat die Dicke eines Proteinmoleküls) oder auch eine Doppelschicht (d. h. hat die Dicke von zwei Proteinmolekülen).

Alternativ liegen die S-Layer-Proteine in der erfindungsgemäßen Struktur in einer geeigneten Flüssigkeit in einer Suspension vor. Insbesondere in wässrigen Lösungen können S-Layer-Proteine stabile Suspension ausbilden. So kristallisieren Monomere in einer Proteinlösung mit der Zeit zu S-Layer-Proteinkristalliten. Bei den entstehenden Kristallen handelt es sich in der Regel um ein- oder doppellagige planare Strukturen, aber auch die Bildung von röhrenartigen Strukturen wird beobachtet. Es entsteht dabei eine heterogene Suspension von verschiedenen S-Layer-Kristallen. Dabei ist die Geschwindigkeit, die Größe und die Art der gebildeten Formen abhängig von zahlreichen Reaktionsparametern, wie Ionenstärke, pH-Wert, Temperatur und Proteinkonzentration. Ein Vorteil ergibt sich dadurch, dass die Proteinmatrix und an sie gebundene Moleküle durch Zentrifugation aus der Suspension als Niederschlag gewonnen werden können.

Nach der Beschichtung der Oberflächen bzw. der Bildung von Suspensionen durch S-Layer-Proteine erfolgt die Synthese von an die Proteine gebundenen Nanopartikeln. Die Ankopplung von Liganden kann zum einen vor der Bildung der Proteinschicht erfolgen. In diesem Fall rekristallisieren die S-Layer-Hybriden und behalten ihre Funktion und Struktur bei. Zum anderen kann eine Ligandenbindung aber auch nach der Beschichtung der Oberflächen bzw. der Bildung der Suspension erfolgen.

Die Erzeugung von Nanopartikeln auf den S-Layer-Proteinen erfolgt bevorzugt durch die Sorption von Metallkomplexen aus einer Salzlösung mit nachfolgender Zugabe eines Reduktionsmittels oder einer basischen Lösung (z.B. Ammoniak).

Die Bindung der organischen Molekülgruppen und/oder Biomoleküle (wie z. B. der Antikörper, Aptamere, organische Fluoreszenzmoleküle oder Enzyme) an das S-Layer-Protein erfolgt bevorzugt kovalent an freie Aminogruppen oder Carboxylgruppen der Aminosäurenseitenketten (Lysin- oder Aspartat- oder Glutamat-Reste) des Proteins. Alternativ erfolgt die Anbindung von Aptameren durch genetische Modifizierung des S-Layer-Proteins, durch Einführung von nukleinsäurebindenden Sequenzen in das Protein, wie z. B. Zinkfingermotiven.

Die Anbindung von Biomolekülen, wie z. B. Antikörpern, Enzymen, fluoreszierenden oder lumineszierenden Biomolekülen erfolgt bevorzugt mittels Crosslinker an freie Aminogruppen oder Carboxylgruppen der Aminosäurenseitenketten (Lysin- oder Aspartat- oder Glutamat-Reste) des Proteins. Alternativ kann diese Anbindung jedoch auch durch die Herstellung eines Fusionsproteins, d. h. durch genetische Modifizierung des S-Layer-Proteins erfolgen.

Die Anbindung von Aptameren oder Antikörpern oder anderen Molekülgruppen, die spezifische Bindungseigenschaften für ein Zielmolekül aufweisen, an die S-Layer-Proteine erfolgt bevorzugt nach der Anbindung der Nanopartikel.

Die Verwendung von Aptameren ermöglicht vorteilhaft die Bindung verschiedenster Zielmoleküle an die Struktur bestehend aus S-Layer-Proteinen und funktionellen Bestandteilen. Dadurch eignen sich die erfindungsgemäßen Strukturen für eine Vielzahl von Anwendungen. So sind zum Beispiel Aptamere bekannt, die an niedermolekulare organische Moleküle, Metallionen, Makromoleküle, Proteine oder sogar an Viruspartikel binden können.

### Bevorzugte Aptamere sind beispielsweise:

| SEQ- ID No. | Sequenz (5' -> 3') | Spezifität |
|---|---|---|
| 1 | ACCTGGGGGAGTATTGCGGAGGAAGGT | AMP (Adenosinmonophosphat) |
| 2 | TGAGGCGGGTGGGTGGGTTGAATATGCTGATTACCCCATCGGA- GAACGTTAAGGCGCTTC | Ethanolamin |
| 3 | CGACCGCAGGTGCACTGGGCGACGTCTCTGGGTGTGGTGT | Oxytetracyclin |
| 4 | CGACGCGCGTTGGTGGTGGATGGTGTGTTACACGTGTTGT | Oxytetracyclin |
| 5 | ACGTTGACGCTGGTGCCCGGTTGTGGTGCGAGTGTTGTGT | Oxytetracyclin |
| 6 | CGAGTTGAGCCGGGCGCGGTACGGGTACTGGTATGTGTGG | Oxytetracyclin |
| 7 | GGGCAGCGGTGGTGTGGCGGGATCTGGGGTTGTGCGGTGT | Oxytetracyclin |
| 8 | GGAGGAACGGGTTCCAGTGTGGGGTCTATCGGGGCGTGCG | Tetracyclin |
| 9 | CGGGAGGGCGGGGTGTGGTATGTATTGAGCGTGGTCCGTG | Tetracyclin |
| 10 | CCCCCGGCAGGCCACGGCTTGGGTTGGTCCCACTGCGCGT | Tetracyclin |
| 11 | GTGCGCACGCTAGGTGGTGATGCTGTGCTACACGTGTTGT | Tetracyclin |
| 12 | GGGGGCACACATGTAGGTGCTGTCCAGGTGTGGTTGTGGT | Tetracyclin |
| [13 | GGGCGGGGGTGCTGGGGGAATGGAGTGCTGCGTGCTGCGG | Tetracyclin |

Durch die erfindungsgemäße Struktur können die aptamergebundenen Partikel detektiert, aus einer Lösung herausgefiltert, abzentrifugiert oder sogar katalytisch gespalten werden.
Die an die Aptamere gebundenen Partikel können vorteilhaft durch Änderung der Salzkonzentration wieder abgelöst werden, so dass die erfindungsgemäße Struktur nach der Verwendung regeneriert und mehrfach verwendet werden kann.

Die vorliegende Erfindung beschreibt die Entwicklung und Verwendung von selbstorganisierenden Hybridmaterialien bestehend aus natürlichen oder gentechnisch veränderten S-Layer-. Proteinen, anorganischen Nanopartikeln und funktionalen organischen Molekülen, wie z. B. Aptameren. Sie basiert auf der Herstellung von strukturiert angeordneten Nanopartikeln und ihrer Kombination mit funktionalen biologischen Molekülen oder organischen Molekülgruppen unter der Verwendung von selbstorganisierenden S-Layer-Proteinen als Matrix und Linker. Die Erfindung beinhaltet weiterhin die Immobilisierung der so hergestellten Nanopartikel-Biomolekül-Hybriden durch die Beschichtung verschiedener Materialien.

Die erfindungsgemäßen multifunktionalen Strukturen ermöglichen zum einen eine Miniaturisierung von Bauteilen, die als Bauteile im Bereich der Elektronik, Optik, Medizin, Katalysatortechnik und Biotechnologie Verwendung finden können. Gleichzeitig werden mit dieser Methode organische Molekülgruppen immobilisiert und auf preisgünstige und unkomplizierte Weise in hochgeordnete Strukturen gebracht.

Die vorliegende Erfindung basiert zum einen auf der Fähigkeit von bakteriellen oder archaealen Hüllproteinen (S-Layer-Proteine), sich selber zu regelmäßigen zweidimensionalen Gitterstrukturen zusammenzulagern. Diese parakristallinen Gitterstrukturen umfassen regelmäßig angeordnete Poren einheitlicher Größe. Diese spezielle Anordnung führt zu regelmäßigen Abstandsbeziehungen zwischen Molekülgruppen des Monomers, Metallclustern oder Molekülgruppen, die durch Modifikation an das Monomer gebunden wurden. Damit stehen mit den S-Layer-Proteinen komplexe selbstorganisierende Strukturen zur Verfügung, die mit technischen Mitteln nicht oder nur sehr aufwendig hergestellt werden können.

Eine weitere Eigenschaft von S-Layer-Proteinen besteht darin, dass sie an Grenzflächen Mono lagen bilden und sich zur Beschichtung von Oberflächen eignen.

Die Kopplung von Molekülgruppen mit Nanopartikeln kann in verschiedenen Bereichen der Elektronik, Optik, Medizin und Biotechnologie Anwendung finden. Durch die Verwendung von S-Layer-Proteinen als Linkermoleküle wird die regelmäßige Anordnung der funktionalisierten Nanopartikel mit definierten Abständen von Molekülgruppe und Nanopartikeln gewährleistet. Zusätzlich können verschiedene Materialien mit den derartig modifizierten S-Layer-Proteinen beschichtet werden. Auf diese Weise können in einem vergleichsweise unkomplizierten und preisgünstigen Verfahren regelmäßige funktionalisierte Nanopartikelstrukturen hergestellt werden.

Aus dem den erfindungsgemäßen Mitteln zugrundeliegendem kombinatorischem Prinzip der Bindung von Nanopartikeln und Liganden bzw. Rezeptormolekülen unter Verwendung von S-Layer-Proteinen als Linker, der Möglichkeit, mit S-Layer Proteinen verschiedene Materialien zu beschichten und damit eine strukturierte Anordnung von ligandengebundenen Nanopartikeln zu erzielen, eröffnen sich Anwendungen, deren Breite den herkömmlichen Mitteln durchweg verschlossen bleibt. Dies betrifft zum einen den Einsatz dieser Hybridmoleküle als Bauteil für Biosensoren. Dabei handelt es sich um Messfühler, die auf der direkten räumlichen Kopplung eines biologischen Systems (des funktionalen Moleküls) mit einem Signalumwandler und einem elektronischen Verstärker basieren. In der vorliegenden Erfindung treten die funktionalen Moleküle in spezifische Wechselwirkung mit den Analyten. Das S-Layer Protein vermittelt die Kopplung mit einem Signalumwandler und gewährleistet eine spezifische räumliche Strukturierung.

In einer vorteilhaften Ausgestaltung der Erfindung werden an der Struktur aus S-Layer-Proteinen Magnetit-Nanopartikel gebunden. Dadurch wird es vorteilhaft ermöglicht, bestimmte Stoffe, die spezifisch an die Aptamere oder Antikörper andere Liganden binden, durch Magnete aus einer Lösung zu entfernen.

In dieser Ausgestaltung der Erfindung liegt die Struktur bevorzugt in einer Suspension in einer wässrigen Lösung vor.

Die Magnetitpartikel haben bevorzugt eine durchschnittliche Partikelgröße von 5 bis 20 nm, besonders bevorzugt 8 bis 15 nm.

Die Herstellung der Nanopartikel erfolgt hier bevorzugt durch Zugabe von S-Layer-Proteinen zu einer wässrigen Lösung, in der sowohl Eisen(II)- und Eisen(III)-Salze gelöst sind und anschließender Erhöhung des pH-Wertes durch Zugabe einer Base, wie z. B. einer Ammoniaklösung. Das Verhältnis Eisen(II)-Salz zu Eisen(III)-Salz in der Lösung beträgt bevorzugt 0,1 : 1 bis 10 : 1, besonders bevorzugt 0,2 : 1 bis 5 : 1.

Im Bereich der medizinischen Therapie ergibt sich ferner die Möglichkeit, Medikamente in die S-Layer-Röhren, welche mit magnetischen Nanopartikeln besetzt sind, einzukapseln. Diese können, im Körper verabreicht, durch induktive Aufheizung der Nanopartikel kontrolliert in den Körper abgegeben werden.

Eine weitere Anwendungsmöglichkeit der S-Layer gebundenen magnetischen Nanopartikel betrifft die Dekontamination von Pathogenen bzw. infektiösen Keimen in Flüssigkeiten. Die Keime lassen sich durch die Anbindung an die Liganden selektiv entfernen und durch eine induktive Aufheizung der magnetischen Partikel abtöten bzw. inaktivieren.

Ein weiteres Einsatzgebiet für S-Layer gekoppelte Magnetpartikel stellt das Gebiet der Separationstechnologie, insbesondere der Affinitätstrenntechnik dar. Die Liganden ermöglichen eine selektive Anbindung von Substraten aus einer Lösung. Durch die Kombination mit magnetischen Nanopartikeln können zum einen diese Substrate nach ihrer Anbindung durch das Anlegen eines magnetischen Feldes aus der Lösung entfernt werden. Dadurch eignet sich dieses Verfahren zur Dekontamination z.B. von Grundwasser aber auch anderen belasteten Wässern.

Zum anderen ermöglicht die Kopplung mit magnetischen Nanopartikeln aber auch die induktive Aufheizung der gebundenen Substrate und damit deren Umwandlung oder auch ihre Elution vom Liganden. Verfahren zur Immobilisierung von Enzymen sind vor allem auf dem Gebiet der Biotechnologie und Verfahrenstechnik von Interesse. Hierbei werden Substrate in einer Lösung durch die Enzyme umgesetzt. Die Immobilisierung der Enzyme durch die Kopplung an S-Layer Proteine und in Kombination mit der Beschichtung von Oberflächen, aber auch die Kopplung mit magnetischen Partikeln ermöglicht die Entfernung der Enzyme aus dem Medium sowie ihre Wiederverwendung nach erfolgtem Umsatz der Substrate.

Ein weiteres Anwendungsgebiet ist die Entwicklung von neuartigen Katalysatoren. Die gekoppelten Liganden ermöglichen eine selektive Anbindung von Zielmolekülen aus einer Lösung. Diese können nachfolgend durch die benachbarten katalytisch aktiven Nanopartikel umgesetzt werden.

In einer weiteren vorteilhaften Ausgestaltung der Erfindung werden an die Struktur Moleküle gebunden, die als Katalysatoren wirken. Besonders bevorzugte Katalysatoren sind dabei Enzyme, oder Nanopartikel, die sowohl als Katalysatoren als auch als Photokatalysatoren wirken können.
Durch die Enzyme oder Nanopartikel werden die mittels Aptameren oder Antikörper an die Struktur gebundenen Zielmoleküle vorteilhaft katalytisch zersetzt. Dies erfolgt z. B. im Falle von photokatalytischen Nanopartikeln nach Anregung durch Licht. Durch die Bestrahlung mir kurzwelligem Licht entstehen an der Oberfläche der Nanopartikel durch die fotokatalytische Spaltung von Wasser Hydroxylradikale, zusätzlich kann ein adsorbiertes Sauerstoffmolekül aktiviert werden und so ein Superoxidion, entstehen. Beide tragen letztendlich zur Oxidation der gebundenen organischen Moleküle bei.
Die S-Layer-Proteine dienen hier als Matrix, welche vorteilhaft die Zielmoleküle und die katalytisch aktiven Substanzen miteinander in Kontakt bringt. Für die Anregung der Photokatalysatoren wird bevorzugt Licht im UV-Bereich verwendet, besonders bevorzugt finden vor allem aber solche Photokatalysatoren Verwendung, die bereits durch diffuses Tageslicht zur Zersetzung der Zielmoleküle angeregt werden können. Dadurch könnten vorteilhaft wässrige Lösungen unabhängig von dem Vorhandensein einer UV-Lichquelle dekontaminiert werden.

Die katalytischen Nanopartikel sind bevorzugt ausgewählt aus Pd-, Pt-, Au- Partikeln und haben bevorzugt eine Partikelgröße von 1-10 nm, besonders bevorzugt 2-8 nm. Die Herstellung erfolgt durch die Sorption von Metallkomplexen aus einer Pt(II), Pd(II) oder Au(III)-Lösung und die nachfolgende Zugabe eines Reduktionsmittels (z.B. H₂, DMAB, NaBH₄).

Die photokatalytischen Nanopartikel sind bevorzugt ausgewählt aus ZnO-, TiO₂- mit oder ohne Dotierungen (z.B. CeO, CoO) Partikeln und haben bevorzugt eine durchschnittliche Partikelgröße von 5 bis 30 nm, besonders bevorzugt 10 bis 25 nm.

Die Herstellung von ZnO-Nanopartikeln erfolgt bevorzugt durch Mischen von S-Layer-Proteinen und Zn(II) in einer wässerigen Zn(II)-Lösung. Die Bildung von S-Layer geträgerten ZnO-Partikeln erfolgt durch die Zugabe einer basischen Lösung, z.B. Ammoniak, NaOH oder Hexamethylentetramin.

Diese Ausgestaltung der Erfindung eignet sich insbesondere zur Reinigung von mit Pharmaka oder anderen organischen Schadstoffmolekülen belastetem Trinkwasser. Grund- oder Trinkwasser sind oftmals kontaminiert durch Arzneimittelrückstände wie Diclofenac, Carbamazepin oder Ibuprofen. Es finden sich desweiteren Rückstände von Antibiotika oder Hormonen, die häufig in der Landwirtschaft zum Einsatz kommen.
Zur Entfernung dieser Schadstoffe werden Aptamere gewählt, die hochspezifisch Pharmaka, organische Schadstoffe wie Antibiotika, Pestizide oder Hormone, Biomoleküle wie z.B. Proteine und Pathogene wie z.B. Viren als Zielmoleküle binden. Gerade Pharmaka wie auch viele andere organische Schadstoffe sind oft schon in sehr geringen Dosen hochwirksame Stoffe, d.h. selbst bei starker Verdünnung besitzen sie immer noch ein hohes Schadstoffpotential. Daher liegen sie oft in gering konzentrierten Lösungen vor und eine zusätzliche Verdünnung findet bei der Einspeisung in das Abwassersystem statt. Dadurch sind herkömmliche Reinigungsmethoden oft unwirksam, da derart niedrig konzentrierte Verunreinigungen nicht entfernt werden können. Durch die hohe Affinität von Aptameren können jedoch auch äußerst niedrig dosierte Verunreinigungen gebunden und entfernt werden.

In einer weiteren vorteilhaften Ausgestaltung der Erfindung enthält die Nanostruktur zusätzlich mindestens zwei zueinander unterschiedliche Moleküle, die zur Erzeugung eines Förster-Resonanz-Energie-Transfers (FRET) geeignet sind. Diese beiden zueinander unterschiedlichen Moleküle werden nachfolgend auch FRET-Donor- und FRET-Akzeptormolekül (oder auch einfach Donor und Akzeptor) genannt. Der Donor und/oder der Akzeptor werden bevorzugt an das S-Layer-Protein und/oder den Ligand (z. B. das Aptamer oder den Antikörper) z. B. kovalent oder durch Koordination gebunden. Wie noch später näher erläutert können jedoch auch Tryptophanreste (oder Tyrosinreste) des Proteins selbst als FRET-Donor dienen, wodurch die Anbindung eines FRET-Donors entfällt.

Der Förster-Resonanz-Energie-Transfer (kurz FRET) ist ein physikalischer Prozess, bei dem Energie eines angeregten Donormoleküles strahlungsfrei auf ein Akzeptormolekül im Abstand von etwa 1,5 bis 10 nm übertragen werden kann. Die Moleküle sind dazu so ausgewählt, dass das Emissionsspektrum des Donormoleküls mit dem Anregungsspektrum des Akzeptormoleküls überlappt. Sind sowohl Donor als auch Akzeptor Fluoreszenzfarbstoffe, spricht man von einem Fluorescence resonance energy transfer. Ist der Donor oder Akzeptor hingegen eine Chemilumineszenz- oder eine Biolumineszenzquelle wird der Begriff Chemiluminescence resonance energy transfer (CRET) bzw. Bioluminescence resonance energy transfer (BRET) verwendet.

Vorteilhaft können durch die Bindung an die S-Layer-Proteine oder die daran gebunden Liganden (z. B. Aptamere oder Antikörper) die nachzuweisenden Moleküle und Atome (Analyte) in die Nähe des Donor/Akzeptorpaares gebracht werden, wodurch das FRET-Signal modifiziert wird und die Änderung messtechnisch erfasst werden kann. Die Anbindung der Analyten erfolgt im Fall von Schwermetallionen durch spezifische Bindung an Aptamere oder durch direkte Bindung an die S-Layer-Proteine. Im Fall von organischen Molekülen oder Makromolekülen wird die Anbindung an den S-Layer bevorzugt durch Liganden, die den Analyten spezifisch binden können, bevorzugt Antikörper oder Aptamere, vermittelt, die zuvor an die S-Layer-Proteine gekoppelt worden waren. Durch Verwendung mehrerer Liganden mit unterschiedlichen Bindungsspezifitäten, die an unterschiedlichen, definierten Stellen des S-Layers gebunden sind, können auch mehrere Analyten zeitgleich erfasst werden.

Die S-Layer-Proteine erlauben dabei vorteilhaft die definierte Anordnung der Donor- und Akzeptormoleküle in dem Abstand von etwa 1,5 bis 10 nm, der für den FRET-Effekt wichtig ist. Die Effizienz dieses Energieübertrags vom Donor auf die Akzeptoren hängt nämlich auf empfindliche Weise vom Abstand, der relativen Orientierung und den elektronischen Übergangsmomenten zwischen Donor und Akzeptor sowie vom Brechungsindex des umgebenden Mediums ab. Verändert sich eine dieser vier Größen durch die Bindung des Analyten, verändert sich die die Effizienz des FRET vom angeregten Donor auf den Akzeptor. Dies lässt sich anhand einer veränderten Spektralverteilung und Intensität der vom Akzeptor emittierten Strahlung nachweisen.

Durch Einsatz von unterschiedlichen Donor/Akzeptorpaaren lassen sich wegen der nanoskaligen Abstände zwischen den Chromophoren FRETs über mehrere Chromophore realisieren. Je nach dem Ort der durch die Anbindung des Analyten verursachten Unterbrechung des FRET wird das emittierte Licht von unterschiedlichen Chromophoren ausgehen. Die spektrale Analyse des Fluoreszenzlichtes trägt damit indirekt Information über den vorwiegenden Ort der Störung. Dies ermöglicht es vorteilhaft, an ein und derselben Matrix den gleichzeitigen Nachweis mehrerer Analyte, sofern sie unterschiedliche Bindungsorte im Netzwerk der energieübertragenden Chromophore besitzen. Solche topologisch unterscheidbaren Bindestellen können durch chemische Synthese oder genetische Modifizierung einer Proteinmatrix realisiert werden. Voraussetzung für diesen ortsabhängigen Prozess ist die feste Lokalisierung der ersten Primäranregung des über Resonanzenergieübertragung gekoppelten Systems. Diese feste Lokalisierung wird dadurch erreicht, dass die Primäranregung in ein definiertes Zentrum erfolgt. Dies kann in S-Layern besonders gut durch ein lumineszentes Übergangsmetall realisiert werden, das im Gegensatz zu den weiteren Chromophoren keine orientierungsabhängige Emission zeigt sondern als nanoskalige lokale Punktstrahlungsquelle an spezifischer Stelle in die Matrix eingebettet ist. Der parallele Nachweis zweier Analyte kann auch durch die Verwendung zweier FRET-Paare (in Verbindung mit zwei verschiedenen Liganden) erfolgen, die sich in ihren Spektren nicht überlappen.

Die Markierung des S-Layer-Proteins erfolgt bevorzugt kovalent an freie Aminogruppen oder Carboxylgruppen der Aminosäurenseitenketten (Lysin- oder Aspartat- oder Glutamat-Reste) des Proteins. Die Anbindung eines Biomoleküls, wie GFP, YFP, CFP und/oder Luciferase erfolgt entweder durch Crosslinking oder durch Herstellung eines Fusionsproteins. Bevorzugt werden in der Erfindung als Donoren und Akzeptoren organische oder anorganische Fluoreszenzfarbstoffe verwendet. Alternativ kann als Akzeptor auch Tryptophan, das als natürlicher Bestandteil der S-Layer-Proteine auftritt, verwendet werden. Lumineszenzquellen können entweder als Akzeptoren oder Donoren eingesetzt werden.

Bevorzugt sind pro Proteinmolekül 1 bis 40, besonders bevorzugt 1 bis 5 Farbstoffmoleküle gebunden.

Die Donor- und Akzeptormoleküle sind dabei entweder bevorzugt auf demselben Proteinmolekül oder auf unterschiedlichen Proteinmolekülen gebunden Um ersteres zu erreichen werden bevorzugt Donor- und Akzeptormoleküle an dasselbe Protein gebunden, z. B. durch die Anbindung an unterschiedliche funktionelle Gruppen, wie z. B. Amino- und Carboxylgruppen, oder eine sequenzielle konzentrationsabhängige Modifizierung gleicher funktioneller Gruppen. Alternativ wird ein Teil der S-Layer-Proteine in Lösung mit dem Donormolekül markiert und der andere Teil mit dem Akzeptormolkül und anschließen beide Teile gemischt, rekristallisiert und so eine gemischte Oberfläche erzeugt.

Die Fluoreszenzfarbstoffe sind bevorzugt ausgewählt aus organischen und anorganischen Farbstoffen. Der Begriff organische Farbstoffe umfasst dabei auch Biomoleküle. Als anorganische Fluoreszenzfarbstoffe werden Europium (bevorzugt Eu³⁺), Cer oder Terbium oder sogenannte Quantum dots (Quantenpunkte), wie z.B. dotierte LaF₃ und LaPO₄-Nanopartikel verwendet. Dazu werden bevorzugt S-Layer-Proteine als Substrat zur Herstellung von anorganischen Fluoreszenzfarbstoffen wie z.B. dotierte LaF₃ und LaPO₄-Nanopartikel verwendet. Alternativ lässt sich z. B. Eu³⁺ effizient über die natürlich im S-Layer vorkommenden Carboxylgruppen von Asparaginsäuren und Glutaminsäuren ohne chemische Syntheseschritte koordinativ anbinden. Diese Gruppen stehen auch dann zur Verfügung, wenn zuvor andere Moleküle, wie Aptamere oder Antikörper chemisch mit dem S-Layer vernetzt werden, da die Anbindung letzterer über andere Aminosäuren (wie z. B. Lysine) erfolgen kann.

In dieser Form dient z. B. bevorzugt Europium (bevorzugt Eu³⁺) als FRET-Donor für Fluorophore, die ein Absorptionsspektrum im roten Spektralbereich besitzen und über 620 nm fluoreszieren. Ein mit einem entsprechenden Chromophor markierter und gleichzeitig Eu³⁺ enthaltender S-Layer stellt damit eine Matrix dar, in der multiple FRET-Paare in regelmäßiger Anordnung vorliegen.

Der Vorteil der Nutzung von Eu³⁺ in dieser Anordnung besteht in der Reduktion des chemischen Syntheseaufwandes, da neben dem spezifitätsvermittelnden Molekül nur ein Chromophor statt eines Chromophorpaares angekoppelt werden muß. Ferner erfolgt die Bindung des gegenüber den beiden anderen Modifikationen kleinen Eu-Ions in definierten Hohlräumen des S-Layers, wodurch mehr reaktive Gruppen für die chemische Modifikation an der Oberfläche zugänglich bleiben als es der Fall bei der Anbindung größerer chemischer Gruppen der Fall ist.

In einer weiteren vorteilhaften Ausgestaltung der Erfindung wird als FRET-Akzeptor ein Fluoreszenzfarbstoff verwendet, dessen Anregungsspektrum mit der Fluoreszenzemission von Tryptophan, das als natürlicher Bestandteil der S-Layer-Proteine auftritt, überlappt. In diesem Fall dienen im Protein vorhandene Tryptophane als FRET-Donor.
Ein bevorzugtes Beispiel für ein solchen FRET-Akzeptor ist Europium (bevorzugt EU³⁺⁾. Erfolgt eine z. B. über Aptamere oder Antikörper vermittelte Anbindung des Analyten, so wird der FRET zwischen den Tryptophanen und dem Europium Eu³⁺ beeinflusst. Bei Anregung von Tryptophan um 280 nm lässt sich die Gegenwart eines Zielmoleküls anhand der auftretenden Lumineszenz von Europium nachweisen. In dieser Ausführung kann ein Analyt mit einer Eigenfluoreszenz im Bereich der Europiumabsorption um 320 nm ohne weitere Kopplung von Farbstoffen nachgewiesen werden. Der chemische Syntheseaufwand beschränkt sich hier auf die Kopplung von spezifitätsvermittelnden Gruppen, wie Aptameren oder Antikörpern.

Bevorzugte Donor-Akzeptorpaare und ihre jeweiligen Anregungsmaxima (Maxₑₓ) sowie Emissionsmaxima (Maxₑₘ) sind:

| **Donor** | | | **Akzeptor** | | |
|---|---|---|---|---|---|
| **Bezeichnung** | **Maxₑₓ** | **Maxₑₘ** | **Bezeichnung** | **Maxₑₓ** | **Maxₑₘ** |
| Alexa Fluor 488 | 495 nm | 519 nm | Alexa Fluor 555 | 555 nm | 565 nm |
| Alexa Fluor 488 | 495 nm | 519 nm | Alexa Fluor 546 | 556 nm | 573 nm |
| Alexa Fluor 488 | 495 nm | 519 nm | Alexa Fluor 568 | 578 nm | 603 nm |
| Alexa Fluor 546 | 556 nm | 573 nm | Alexa Fluor 633 | 632 nm | 647 nm |
| Alexa Fluor 555 | 555 nm | 565 nm | Alexa Fluor 647 | 650 nm | 668 nm |
| Alexa Fluor 568 | 578 nm | 603 nm | Cy5 | 649 nm | 670 nm |
| Alexa Fluor 568 | 578 nm | 603 nm | Alexa Fluor 633 | 632 nm | 647 nm |
| Alexa Fluor 568 | 578 nm | 603 nm | Alexa Fluor 647 | 650 nm | 668 nm |
| Alexa Fluor 568 | 578 nm | 603 nm | Cy5 | 649 nm | 670 nm |
| zyan fluoreszierendes Protein (CFP) | 452 nm | 505 nm | gelb fluoreszierendes Protein (YFP) | 514 nm | 527 nm |
| grün fluoreszierendes Protein (GFP) | 395 nm | 509 nm | Rhodamin 123 | 507 nm | 529 nm |
| Fluoresceinisothiocyanat (FITC) | 494 nm | 518 nm | Cy3 | 550 nm | 570 nm |
| Europium (Eu³⁺) | 395 nm 466 nm | 617 nm | Allophycocyanin. | 650 nm | 660 nm |
| Luciferase | | | YFP | 514 nm | 527 nm |
| FITC | 494 nm | 518 nm | TRITC | 550 nm | 570 nm |
| Cy3 | 550 nm | 570 nm | Cy5 | 649 nm | 670 nm |
| Cy3 | 550 nm | 570 nm | Alexa Fluor 633 | 632 nm | 647 nm |
| Cy3 | 550 nm | 570 nm | Alexa Fluor 647 | 650 nm | 668 nm |
| EGFP | 488 nm | 509 nm | Cy3 | 550 nm | 570 nm |
| EGFP | 488 nm | 509 nm | YFP | 514 nm | 527 nm |
| Fluorescein | 494 nm | 518 nm | TRITC | 550 nm | 570 nm |
| Fluorescein | 494 nm | 518 nm | Cy3 | 550 nm | 570 nm |
| Eu³⁺ (oder LaF₃) | 395 nm | 591 nm | Alexa Fluor 594 (oder auch Alexa Fluor 610 oder 633) | 590 nm | 630 nm |
| LaPO₄ (oder Ce oder Tb) | 266 nm | 542 nm | Alexa Fluor 532 | 530 nm | 560 nm |
| Proteineigenes Trp | 280 nm | 353 nm | Eu³⁺ | 395 nm 466 nm | 617 nm |
| Tyr | 274 nm | 303 nm | Trp | 280 nm | 353 nm |

Ein weiterer Gegenstand der Erfindung ist daher ein Sensor gemäß Anspruch 7, der S-Layer-Proteine und zwei unterschiedliche Moleküle, die zur Erzeugung eines Förster-Resonanz-Energie-Transfer (FRET) geeignet sind, enthält, wobei die S-Layer-Proteine eine kristalline Schicht auf einer Oberfläche eines Substrats ausbilden oder in einer geeigneten Flüssigkeit als Suspension vorliegen, wobei an die selbstorganisierenden Proteine Aptamere und/oder Antikörper gebunden sind, wobei durch die Bindung an die Aptamere oder Antikörper die nachzuweisenden Moleküle und/oder Atome in die Nähe des Donor/Akzeptorpaares gebracht werden, wodurch das FRET-Signal modifiziert und die Änderung messtechnisch erfasst wird.

Der Sensor eignet sich insbesondere:
a.) zum Nachweis von Schwermetallen und
b.) zum Nachweis von Arzneimittelmolekülen oder organischen Molekülen, insbesondere Schadstoffmolekülen.

Das S-Layer-Protein, das Substrat, der FRET-Akzeptor und alternative FRET-DonorAkzeptor-Paare sind bevorzugt wie oben beschreiben ausgewählt.

Die selbstorganisierenden Proteine sind S-Layer-Proteine, da diese viele Schwermetalle unspezifisch binden können, wie zum Beispiel Aluminium, Cadmium oder Blei.

Besonders bevorzugt sind S-Layer-Proteine, die von Bakterien aus schwermetallhaltigen Umgebungen, wie zum Beispiel Uranabraumhalden, stammen. Diese Bakterien gehören bevorzugt zu den Gattungen *Lysinibacillus* und *Bacillus* (z.B. *Lysinibacillus sphaericus* JG-A12, *Bacillus* sp. JG-B12). Diese S-Layer-Proteine können aufgrund ihrer speziellen Struktur Schwermetalle wie Uran spezifisch binden, ohne dazu zusätzliche spezifische Bindungsmoleküle zu benötigen.

Durch die Bindung von Schwermetallen an den S-Layer kann eine Veränderung des FRET detektiert werden.

Zum spezifischen Nachweis von Arzneimittelmolekülen oder organischen Molekülen oder auch Schwermetallen sind an die S-Layer-Proteine Aptamere oder Antikörper gebunden. Diese werden bevorzugt wie oben beschrieben an die Proteinschicht gebunden. In diesem Falle können der FRET-Akzeptor und/oder FRET-Donor auch an den Liganden gebunden sein.

Durch diese zusätzliche Kopplung von Aptameren oder Antikörpern wird vorteilhaft eine hohe Spezifität erreicht.

Durch die Bindung des nachzuweisenden Zielmoleküls an das Aptamer oder den Antikörper kommt es zur Veränderung optisch relevanter Parameter, wie z.B. sterische Behinderung des FRET-Akzeptors oder Änderung des Brechungsindexes, was die Effizienz des präexistenten FRET-Signals beinflusst und spektroskopisch nachweisbar ist.

In beiden Fällen kann das S-Layer-Protein wie oben beschrieben in Suspension vorliegen oder eine (oder mehrere) Schicht(en) auf einem Substrat bilden. Das Substrat ist dabei bevorzugt wie oben beschrieben ausgewählt. Derartige Schichten können auf transparenten Substraten als Trägern, wie zum Beispiel Glas oder MgO immobilisiert werden und in eine Durchflusskammer eingebaut werden. Alternativ kann das Protein auch wie oben beschrieben in einer Nanostruktur (z. B. in Form der beschriebenen Magnetitpartikel) vorliegen.

Der Sensor enthält bevorzugt als weitere Bestandteile eine Strahlungsquelle, eine Sendeoptik zum Leiten des Lichts auf die Proteinsuspension, die Nanostruktur oder die Proteinschicht (Anregung des Donors), einer Empfangsoptik zum Leiten des Lichts sowie einem Detektor (zur Messung der Emission, z. B. zur Detektion der Modulationen des präexistenten FRET).

Der Detektor ist ein geeignetes Fluoreszenzauslesegerät, wie z. B. ein Fluoreszenzmikroskop oder ein Array-Reader.

Zum Schutze der Optik kann diese auf der nicht modifizierten Seite des transparenten Trägers realisiert werden. Die Bindung eines Analyten bedingt dann eine zunehmende Modulation des FRET-Signals, welches ab einem bestimmten Schwellenwert detektierbar wird. Die hochregelmäßige Anordnung der Farbstoffe führt dabei zu einer Verstärkung des Signals.

Die Biosensoren können dabei vorteilhaft auch so ausgestaltet werden, dass sie den parallelen spezifischen Nachweis verschiedener Analyten innerhalb einer Testsubstanz ermöglichen.

Dazu werden vorzugsweise in verschiedenen Koordinaten des Trägers Aptamere oder andere Liganden mit unterschiedlichen Bindungsspezifitäten aufgebracht. Dies geschieht bevorzugt, indem zunächst unterschiedliche Liganden an voneinander getrennte Proben des S-Layer-Proteins in Suspension gekoppelt werden und diese markierten Proteine dann in unterschiedlichen Koordinaten in Form eines Arrays aufgetragen werden. Die Anzahl der parallel nachzuweisenden Analyte kann auch durch die Verwendung zweier unterschiedlicher FRET-Paare (in Verbindung mit verschiedenen Liganden) erhöht werden. Die FRET-Paare werden dabei so ausgewählt, dass sich die Anregungsspektren und Emissionsspektren nicht überlappen.

### Ausführungsbeispiele:

Anhand der folgenden Figuren und Ausführungsbeispiele soll die Erfindung näher erläutert werden ohne die Erfindung auf diese zu beschränken.
**Figur 1** zeigt verschiedene Kristallisationssymmetrien von S-Layem: schräg (Fig. 1a, b), quadratisch (Fig. 1c) oder hexagonal (Fig. 1d, e).

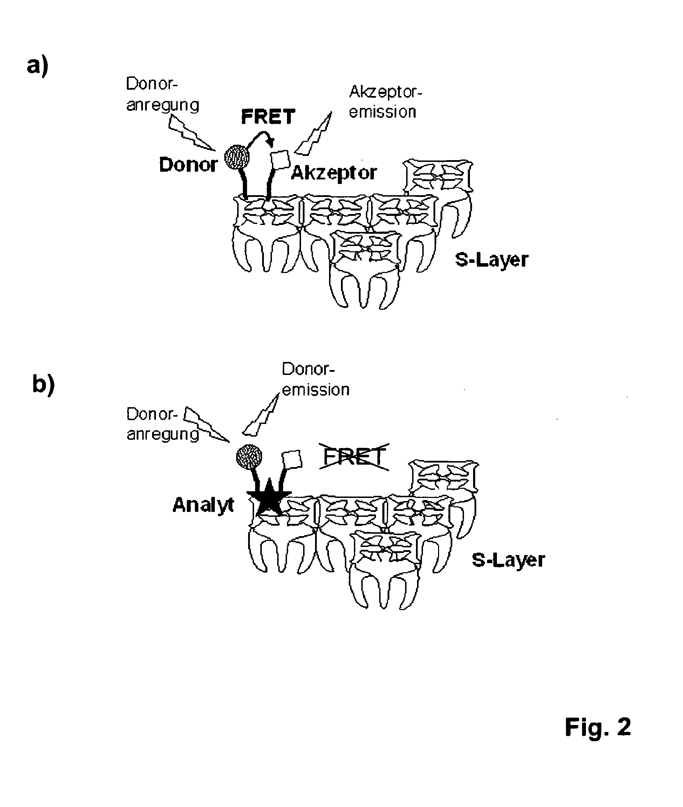

**Figur 2** illustriert das Prinzip der Modulation eines präexistenten FRET durch die Bindung eines Analyten an den S-Layer. a) An den S-Layer gekoppelt sind zwei Fluoreszenzfarbstoffe als Donor (dargestellt durch eine schraffierte Kugel) und Akzeptor (dargestellt durch ein weißes Quadrat). Der Donor wird durch Licht einer geeigneten Wellenlänge angeregt, und die Anregungsenergie geht durch FRET auf den Akzeptor über, der Licht einer spezifischen Wellenlänge emittiert. b) Durch die Bindung des Analyten (dargestellt durch einen schwarzen Stern) wird der FRET verhindert, der Akzeptor emittiert kein Licht. Der Donor gibt die Anregungsenergie durch Emission von Licht, das jedoch eine andere Wellenlänge aufweist, als das vom Akzeptor abgegebene.

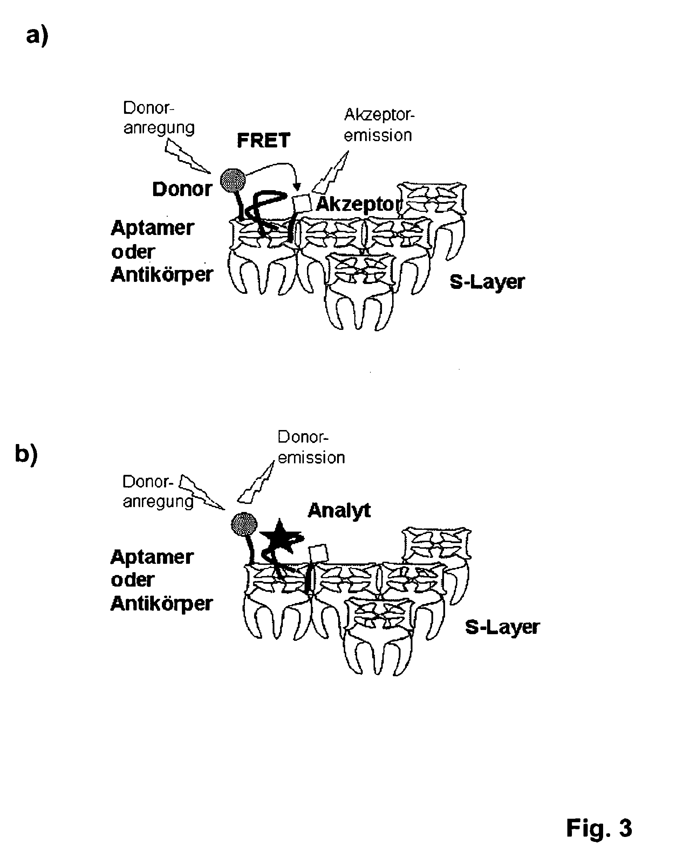

**Figur 3** zeigt ebenfalls das Prinzip der Modulation eines präexistenten FRET durch die Bindung eines Analyten. Allerdings erfolgt hier die Bindung des Analyten nicht direkt an den S-Layer, sondern an ein Molekül mit spezifischen Bindungseigenschaften, wie ein Aptamer oder ein Antikörper (symbolisiert durch eine schwarze Schlaufe).

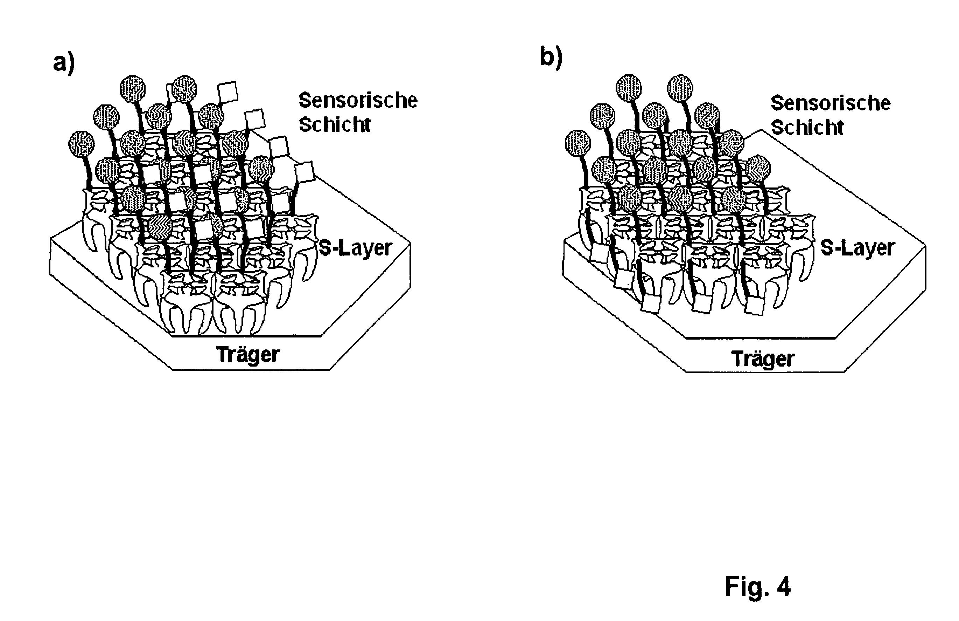

**Figur 4** illustriert den Aufbau eines erfindungsgemäßen Biosensors. Die erfindungsgemäße Nanostruktur wird auf einem transparenten Träger, wie zum Beispiel Glas oder MgO immobilisiert. Der Sensor setzt sich zusammen aus einer Strahlungsquelle, einer Sendeoptik zum Leiten des Lichts auf die Proteinschicht (Anregung des Donors), einer Empfangsoptik zum Leiten des Lichts sowie einem Detektor (Detektion der Modulationen des präexistenten FRET). Zum Schutze der Optik kann diese auf der nicht modifizierten Seite des transparenten Trägers realisiert werden. Figur 2 a) und b) zeigen, wie die an den S-Layer gebundenen Akzeptoren und Donoren an unterschiedlichen Orten auf dem S-Layer lokalisiert sein können.

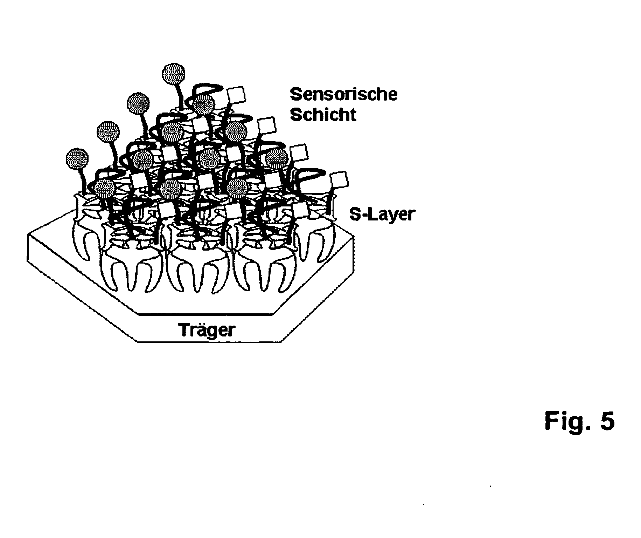

**Figur 5** zeigt den Aufbau eines erfindungsgemäßen Biosensors aus Träger, S-Layer, Donor (schraffierte Kugel), Akzeptor (weißes Quadrat) und spezifisch bindendem Liganden (Aptamer oder Antikörper).
**Figur 6** illustriert das Prinzip des Nachweises von (Schwer-)Metallionen durch Bindung an die Schicht aus S-Layer-Proteinen und die dadurch verursachte Veränderung eines präexistenten FRET-Signals.

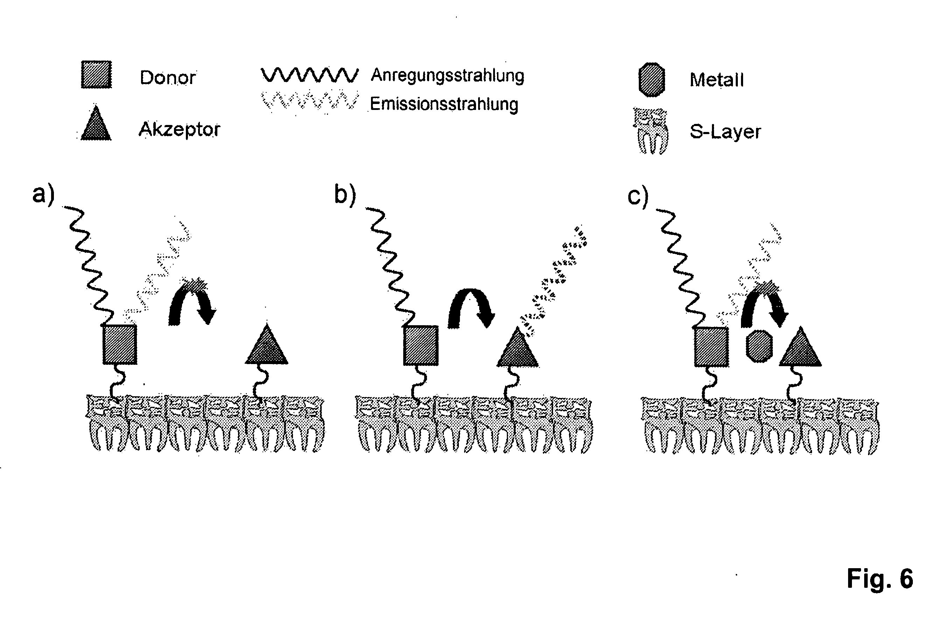

Figur 6 a) zeigt schematisch, dass die zwei als Donor (dargestellt durch ein Quadrat) und Akzeptor (dargestellt durch ein Dreieck) verwendeten Fluoreszenzfarbstoffe nur dann einen FRET produzieren können, wenn sie nicht zu weit voneinander entfernt sind. Bei zu großen Abständen (mehr als ca. 10 nm) kommt es zu keiner Übertragung der Anregungsenergie von Donor zu Akzeptor. Sind Donor und Akzeptor jedoch nahe zueinander (Figur 6 b), wird die Anregungsenergie durch FRET vom Donor strahlungsfrei auf den Akzeptor übertragen und der Donor emittiert Licht mit seiner spezifischen Emissionswellenlänge.
Figur 6 c): Durch die Bindung von (Schwer-)Metallionen (dargestellt durch ein Sechseck) wird der FRET gestört, d. h. es wird keine Energie mehr auf den Akzeptor übertragen und dieser emittiert kein Licht. Der Donor gibt die Anregungsenergie durch Emission von Licht ab, das jedoch eine andere Wellenlänge aufweist, als das vom Akzeptor abgegebene.

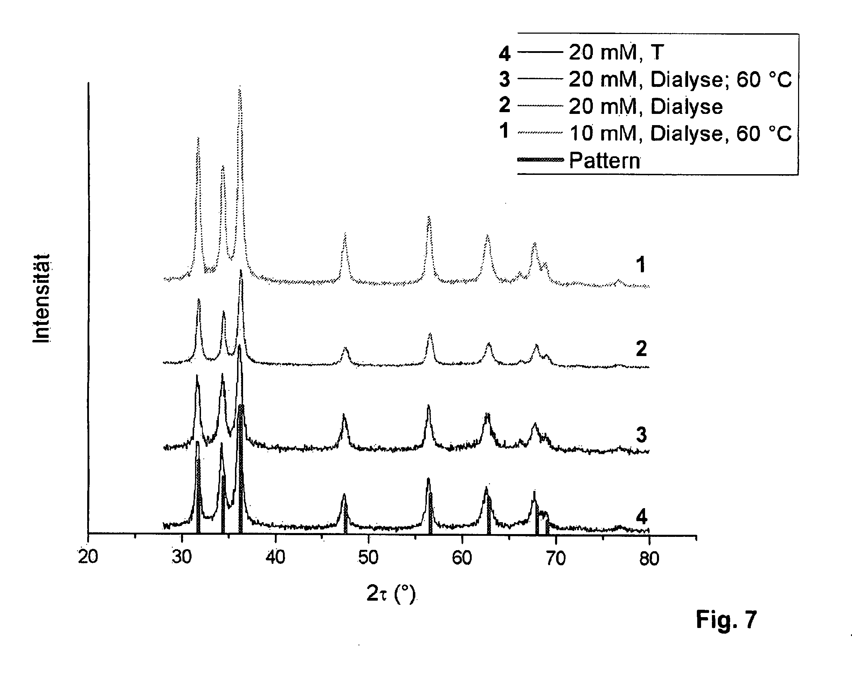

### Figuren 7 bis 11 werden nachfolgend im Text erläutert.

### Ausführungsbeispiel 1a: Magnetitpartikel und Markierung mit Aptameren

Zu einer Lösung bestehend aus Fe(III) und Fe(II)-Salzlösungen mit variierenden molaren Verhältnissen (2:1, 0,5:1 bis 4:1) werden gereinigte S-Layer Proteine, bevorzugt der Gattung *Bacillus* oder *Lysinibacillus,* in variierender Konzentration (bevorzugt 0,5-5 mg/ml) zugegeben. Die Bildung von Magnetitpartikeln erfolgt durch die anschließende Zugabe einer 25%igen Ammoniaklösung. Die entstehenden Magnetitpartikeln besitzen eine Größe von 10-12 nm und sind an die S-Layer-Proteine gebunden.
Nachfolgend werden an die S-Layer-Proteine Aptamere gebunden, die spezifisch organische Moleküle oder auch Metallionen aus einer wässerigen Lösung binden (z.B. AMP bindendes Aptamer mit der Sequenz: ACCTGGGGGAGTATTGCGGAGGAAGGT, Nishihira et al. 2004 Nucl. Ac. Sympos. Ser. 48, pp. 135-136, oder Ethanolamin bindendes Aptamer mit der Sequenz z.B. TGAGGCGGGTGGGTGGGTTGAATATGCTGATTACCCCATCGGAGAACGTTAAGGCGCTTC, D. Mann et al. 2005 Biochemical and Biophysikal Research Communications 338, 1928-1934).
Durch das Anlegen eines magnetischen Feldes können die S-Layer gebundenen MagnetitPartikel zusammen mit den gebundenen organischen Molekülen oder Metallionen aus dem Medium entfernt werden.

Weitere bevorzugte Aptamersequenzen sind:
1.) für Oxytetracyclin:
   5'-CGACCGCAGGTGCACTGGGCGACGTCTCTGGGTGTGGTGT-3'
   5'-CGACGCGCGTTGGTGGTGGATGGTGTGTTACACGTGTTGT-3'
   5'-ACGTTGACGCTGGTGCCCGGTTGTGGTGCGAGTGTTGTGT-3'
   5'-CGAGTTGAGCCGGGCGCGGTACGGGTACTGGTATGTGTGG-3'
   5'-ACGTTGACGCTGGTGCCCGGTTGTGGTGCGAGTGTTGTGT-3'
   (Niazi JH, Lee SJ, Kim YS, Gu MB.ssDNA aptamers that selectively bind oxytetracycline Bioorg Med Chem. 16(2008)3 S. 1254-61)
2.) für Tetracyclin:
   5'-GGGCAGCGGTGGTGTGGCGGGATCTGGGGTTGTGCGGTGT-3'
   5'-GGAGGAACGGGTTCCAGTGTGGGGTCTATCGGGGCGTGCG-3'
   5'-CGGGAGGGCGGGGTGTGGTATGTATTGAGCGTGGTCCGTG-3'
   5'-CCCCCGGCAGGCCACGGCTTGGGTTGGTCCCACTGCGCGT-3'
   5'-GTGCGCACGCTAGGTGGTGATGCTGTGCTACACGTGTTGT-3'
   5'-GGGGGCACACATGTAGGTGCTGTCCAGGTGTGGTTGTGGT-3'
   5'-GGGCGGGGGTGCTGGGGGAATGGAGTGCTGCGTGCTGCGG-3
   (Niazi JH, Lee SJ, Gu MB. Single-stranded DNA aptamers specific for antibiotics tetracyclines. Bioorg. Med. Chem. 16(2008)15 S. 7245-53).

### Ausführungsbeispiel 1b: Beschichtung eines Substrats mit S-Layer-Proteinen

Die Herstellung einer S-Layer Schicht auf einem Substrat erfolgt zum einen durch die Adsorption eines nativen Proteins auf einem Substrat, das modifiziert sein kann. Alternativ kann eine Rekristallisation des mit Guanidinhydrochlorid denaturierten S-Layer Proteins nach einer Entfernung des Guanidinhydrochlorids durch die Dialyse gegen eine CaCl₂-Lösung (5-20 mM) erfolgen. Die Proteinkonzentration beträgt bei dieser Methode 0,5-5 mg/ml. Als Substrate dienen z.B. Glas, MgO, Glimmer, SiO₂, Si, Al₂O₃. Diese können silanisiert oder mit einer Schicht aus anionischen oder kationischen Polymeren oder sekundären Zellwandpolymeren (SCWPs) modifiziert sein, um eine stabile Beschichtung zu begünstigen. Alternativ ist eine Modifizierung der Oberfläche mit z.B. Aminogruppen (Aminierung, Wieringa, R. H., Schouten, A. J., 1996, Macromolecules 29, pp. 3032-3034) denkbar. In diesem Fall werden die S-Layer Proteine mit dem Substrat durch Crosslinking des Proteins mit den Aminogruppen kovalent verknüpft.

Unterschiedliche Substrate wurden mit der oben beschriebenen Rekristallisation-Methode erfolgreich mit S-Layer-Proteinen (aus *L. sphaericus* JG-A12) beschichtet. Die Ergebnisse sind in der nachfolgenden Tabelle zusammengefasst:

| Substrat | mg S-Layer/g Substrat |
|---|---|
| nanoporöses Silikat (SBA 15) | 53 |
| mesoporöses Silikat (MCM 41) | 116 |
| Aluminiumoxid (Puralox) | 52 |
| Blähton (Baumarkt) | 62 |
| Partikel Ø < 0,63 mm | |
| Industrielle Ton-Keramik | 56 |
| Partikel Ø < 0,63 mm | |
| Glasschaum (TECHNOpor) | 56 |
| Partikel Ø < 0,63 mm | |
| Silicagel G 62 | 68 |
| Carbonanotubes CNT (Sigma-Aldrich) | 168 |
| Carbonanotubes CNT C 150 HP (Bayer) | 67 |

Der Verlauf der Beschichtung wurde durch Atomkraftmikroskopie (AFM) überwacht, gebundene Proteinmengen über eine Proteinbestimmung quantifiziert und der Erfolg der Beschichtung zusätzlich durch Floureszenzmarkierung der zur Beschichtung verwendeten S-Layer-Protiene überprüft.

### Ausführungsbeispiel 2: Katalytische Partikel

### a) ZnO-Nanopartikeln als Photokatalysatoren

Zu einer wässerigen Zn(II)-Lösung werden gereinigte S-Layer-Proteine gegeben. Die Bildung von S-Layer geträgerten ZnO-Partikeln erfolgt durch die Zugabe einer basischen Lösung (z.B. NH₃, NaOH, Hexamethylentetramin) unter Rühren bei Raumtemperatur. Der entstehende Niederschlag wird gegen H₂O bei Raumtemperatur dialysiert und/oder erhitzt (50-80 °C). Die entstehenden ZnO-Partikel besitzen eine Größe von 12-23 nm und sind an die S-Layer Proteine gebunden.

In **Figur 7** werden verschiedene Prozessparameter bei der Herstellung von ZnO-Nanopartikel auf S-Layern verglichen. Verglichen werden unterschiedliche Konzentrationen von ZnCl₂ und die Dialyse bei Raumtemperatur (keine Temperaturangabe in Fig. 7) und 60 °C.
Es entstehen einheitlich Partikel mit einem Durchmesser von 14 nm (↔ 23 nm ohne S-Layer).

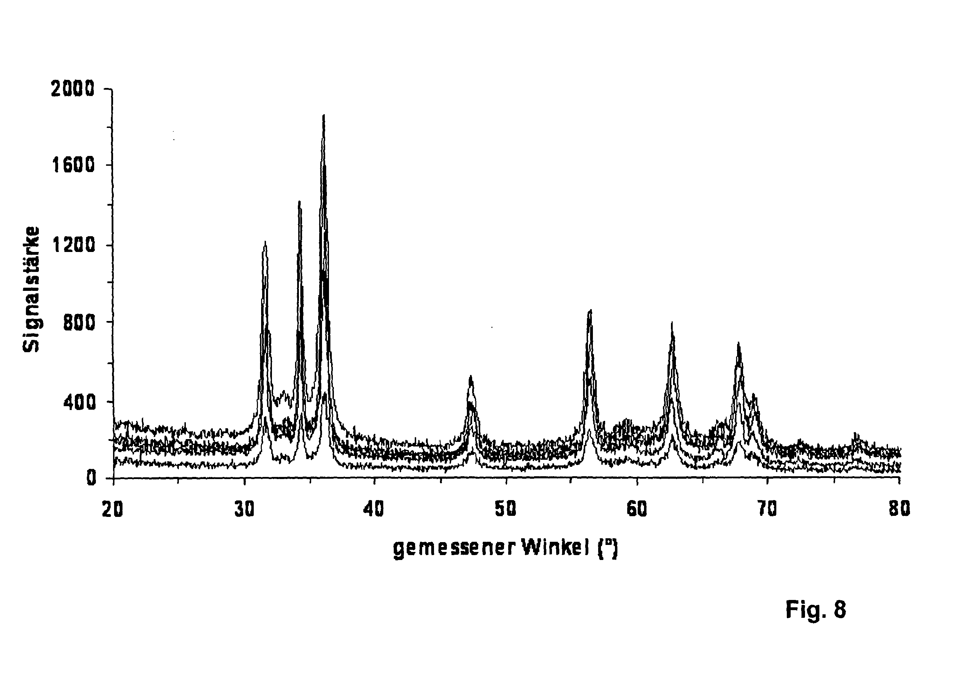

**Figur 8** zeigt XRD-Spektren der ZnO-Nanopartikel auf S-Layern verschiedener Stämme. Die Analysen bestätigen die Bildung verschieden großer aber kristalliner ZnO-Nanopartikel.

Neben der Herstellung von reinen ZnO-Partikeln ist auch die Herstellung von dotierten ZnO-Partikeln denkbar, wodurch die photokatalytischen Eigenschaften verbessert werden. Die Dotierung erfolgt durch eine Mischung der Zn(II)-Lösung mit einer weiteren Metallsalzlösung (z.B. Ce(II), Co(II), Cu(II)) in variierendem Mischungsverhältnis.

Die Anbindung eine Aptamers an die so hergestellte mit Nanopartikeln beladene Proteinschicht erfolgt, wie in Ausführungsbeispiel 1a beschrieben.

Die so hergestellten Verbundmaterialien, bestehend aus Trägermaterial, S-Layer-Protein, photokatalytisch aktiven Nanopartikeln und Aptameren dient zum einen als Filtermaterial. Aus einer wässerigen Lösung werden durch die Aptamere spezifisch organische Moleküle gebunden. Damit werden diese Moleküle in eine räumliche Nähe zu den photokatalytisch aktiven Nanopartikeln gerückt. Durch die Bestrahlung mit kurzwelligem Licht (im UV-Bereich, idealerweise aber auch diffuses Tageslicht) werden an der Oberfläche der Nanopartikel Sauerstoffmoleküle und Wasser aktiviert, die die gebundenen organischen Moleküle oxidieren.

### b) TiO₂-Nanopartikel als Photokatalysatoren

Die Herstellung von TiO₂-Partikeln erfolgt nach Patent DE 19941448. Lösungen von TiF₃ und Ti(III)-ethylenglycolat werden in einer NaCl-Lösung (2 mol/L) bei 0°C unter Argon als Schutzgas gelöst. Zu dieser Lösung wird gereinigtes S-Layer-Protein gegeben. Danach wird die Temperatur der Lösung innerhalb von 10 min auf 30°C linear gesteigert. Die Abscheidereaktion wird in O₂-gesättigter Lösung hergestellt, während der pH-Wert der Lösung bei 6,2-6,4 gehalten wird.

Die Herstellung einer S-Layer Schicht auf einem Substrat erfolgt wie in Ausführungsbeispiel 1b und die Anbindung eine Aptamers an die so hergestellte mit Nanopartikeln beladene Proteinschicht erfolgt wie in Ausführungsbeispiel 1a beschrieben.

Die so hergestellten Verbundmaterialien, bestehend aus Trägermaterial, S-Layer-Protein, photokatalytisch aktiven Nanopartikeln und Aptameren dient zum einen als Filtermaterial, zum anderen als Photokatalysator. Aus einer wässerigen Lösung werden durch die Aptamere spezifisch organische Moleküle gebunden. Damit werden diese Moleküle in eine räumliche Nähe zu den photokatalytisch aktiven Nanopartikeln gerückt. Durch die Bestrahlung mir kurzwelligem Licht werden an der Oberfläche der Nanopartikel Sauerstoffmoleküle und umgebendes Wasser aktiviert, die die gebundenen organischen Moleküle oxidieren.

### c) Herstellung von Pt- und Pd-Clustern sowie von bimetallischen Gold-Palladium oder Platin-Palladium Clustern als Katalysatoren

Bakterielle S-Layer Proteine werden als Matrix zur Herstellung von hochgeordneten Nanopartikel-Strukturen verwendet. Dieses Verfahren wird im Patent DE 10204532 am Beispiel von Pd und Pt-Partikeln beschrieben. Hierbei werden isolierte S-Layer-Proteine in einer wässerigen Pd(II)- oder Pt(II)-Salzlösung inkubiert. Die gebundenen Metallkomplexe werden durch die Zugabe eines Reduktionsmittels (wie z. B. NaN₃, DMAB, H₂, NaBH₄) reduziert. Die Darstellung von S-Layer geträgerten Au- oder Pt-Partikel erfolgt wie in Ausführungsbeispiel 1a oder Ausführungsbeispiel 1b beschrieben. Diese Nanopartikel dienen als Nucleationskeime bei der Reduktion einer wässerigen Na₂PdCl₄-Lösung mit einer wässerigen Lösung des Trinatriumsalzes der Citronensäure bei 70°C (Patent DE 19930893 A1) oder alternativ durch die Zugabe von DMAB oder NaBH₄.

Die Herstellung einer S-Layer Schicht auf einem Substrat erfolgt wie in Ausführungsbeispiel 1b beschrieben. Die Anbindung eine Aptamers an die so hergestellte mit Nanopartikeln beladene Proteinschicht erfolgt wie in Ausführungsbeispiel 1a beschrieben.

Die so hergestellten Verbundmaterialien, bestehend aus Trägermaterial, S-Layer-Protein, katalytisch aktiven Nanopartikeln und Aptameren dient als Katalysator. Aus einer wässerigen Lösung werden durch die Aptamere spezifisch organische Moleküle gebunden. Damit werden diese Moleküle in eine räumliche Nähe zu den katalytisch aktiven Nanopartikeln gerückt und katalytisch gespalten.

### Ausführungsbeispiel 3: FRET mit kovalent gekoppelten Farbstoffen

### a) FRET mit organischen Molekülen als Donor und Akzeptor

Die verwendeten Fluoreszenzfarbstoffe FITC und TRITC wurden von der Firma Molecular Probes bezogen. Beide Fluoreszenzfarbstoffe wurden zunächst in DMSO gelöst, sodass sich eine Konzentration von 1 mg/ml ergab. Die Anbindung der Farbstoffe an das S-Layer Protein erfolgte über freie Aminogruppen (Lysinreste) bzw. freie Carboxylgruppen (Asp und Glu-Reste). Dazu wurde EDC (1-Ethyl-3-(3-dimethylaminopropyl)carbodürnid) im 200fachen molaren Überschuss zum jeweiligen Reaktionspartner gegeben und so dessen Carboxylgruppen aktiviert. Bei allen Versuchen war ein Phosphatpuffer pH 5,4 das Lösungsmittel. Es wurden für jeden Ansatz 1 mg Protein in 1 ml Phosphatpuffer gelöst. Dieser Proteinlösung wurde dann der jeweilige Fluoreszenzfarbstoff im molaren Verhältnis 2:1 (Farbstoff:Protein) zugegeben und der Ansatz gemischt. Die Ansätze wurden für 24 h bei Raumtemperatur im Dunkeln inkubiert. Anschließende wurde nicht reagierter Farbstoff durch mehrmaliges Filtrieren mittels Ultrafiltration entfernt.

Die Herstellung einer S-Layer Schicht auf einem Substrat erfolgt wie in Ausführungsbeispiel 1a oder Ausführungsbeispiel 1b beschrieben; die Anbindung eine Aptamers an die so hergestellte Proteinschicht erfolgt wie in Ausführungsbeispiel 1a beschrieben.

Die Emissions- und Extinktionsmessungen erfolgten mit dem Fluoreszenzspektrometer LS 55 (LS 55 Fluoreszenzspektrometer, 230 V, Perkin Elmer). Als Anregungswellenlängen wurden 408, 460, 488 und 530 nm gewählt. Dabei dienten die ersten drei Wellenlängen der Anregung des Donors und die Wellenlänge 530 nm der Anregung des Akzeptors. Die Messung der Extinktion erfolgte bei den Wellenlängen 520 und 610 nm, wobei die Anregung bei 400 nm startete.

### b) FRET mit einem Donor/Akzeptor-Paar bestehend aus einem anorganischen und einem organischen Fluoreszenzfarbstoff

Alternativ werden S-Layer-Proteine als Substrat zur Herstellung von anorganischen Fluoreszenzfarbstoffen wie z.B. dotierte LaF₃- und LaPO₄-Partikel verwendet. Für die Herstellung der LaF₃- bzw. LaPO₄-Partikel werden die gereinigten und rekristallisierten S-Layer Proteine (z.B. 0,5 mg/ml) in einer Lösung von z.B. 20 mM La(III) inkubiert und mit einer Lösung von F⁻ oder PO₄⁻ versetzt, bis ein deutlicher Niederschlag erfolgt. Eine Dotierung z.B. mit Eu oder Ce aber auch Metallen wie Co, Cu kann durch die die Zugabe z.B. einer Eu(III) oder Ce(III)-Lösung variierender Konzentration erfolgen, verbunden mit einer Kopräzipitation bei der Zugabe von der NH₄F bzw. PO₄³⁻ Lösung. Die hergestellten Partikel haben eine Partikelgröße von 10-30 nm, bevorzugt von 12-20 nm.

Die Herstellung einer S-Layer Schicht auf einem Substrat erfolgt wie in Ausführungsbeispiel 1a oder Ausführungsbeispiel 1b beschrieben; die Anbindung eine Aptamers an die so hergestellte Proteinschicht erfolgt wie in Ausführungsbeispiel 1a beschrieben. Die Extinktions- und Emissionsmessungen wurden wie in Ausführungsbeispiel 3a beschrieben durchgeführt.

### Ausführungsbeispiel 4: genetische Modifizierung von S-Layer-Proteinen

Durch genetische Modifizierung können in das S-Layer Protein DNA-bindende AminosäureSequenzen eingeführt werden. Diese Aminosäuresequenzen, auch Zinkfinger oder Zn-Finger genannt, erkennen und binden spezifische DNA-Motive, z.B. Zif268 (Kim et al., Proc. Natl. Acad. Sci USA, 1998; 95(6):2812-2817), NRE (Kim et al., Proc. Natl. Acad. Sci USA, 1998;95(6):28 12-2817), Sp1C (Wolfe et al., Annu. Rev. Biophys. Biomol. Struct. 2000;29: 183-212). Die Einführung derartiger DNA-bindender Sequenzen ermöglicht die Anbindung von Oligonucleotiden insbesondere Aptameren an das S-Layer-Protein.

### Ausführungsbeispiel 5: Anwendung von ZnO-Nanopartikel zum Diclofenac-Abbau

In diesem Anwendungsbeispiel werden die in Ausführungsbeispiel 2a mit S-Layerproteinen hergestellten photokatalytischen ZnO-Nanopartikel verwendet um das Pharmakon Diclofenac (2-((2,6-Dichloranilino)phenyl)acetat) abzubauen. Verwendet wird dazu eine wässrige Lösung von 100 µM Diclofenac. Der Abbau von Diclofenac wird mittels HPLC gemessen.

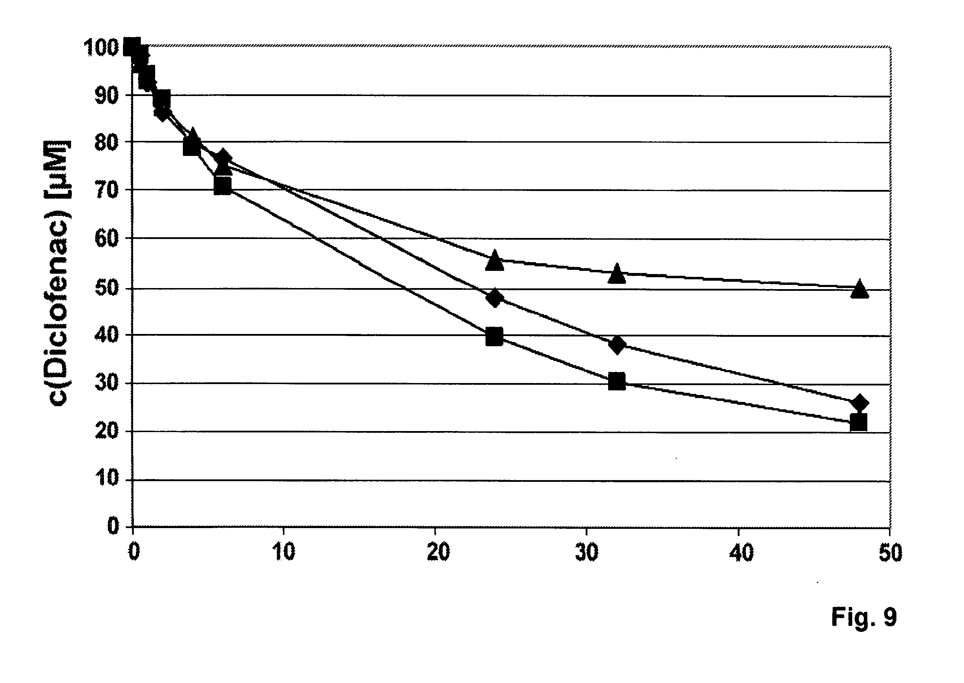

**Figur 9** zeigt den Diclofenac-Abbau durch 1 mg ZnO-Nanopartikel ohne S-Layer (▲), hergestellt mit 30 mg S-Layer (◆) und hergestellt mit 10 mg S-Layer (■) des Stammes *Lysinibacillus sphaericus* JG-A12.

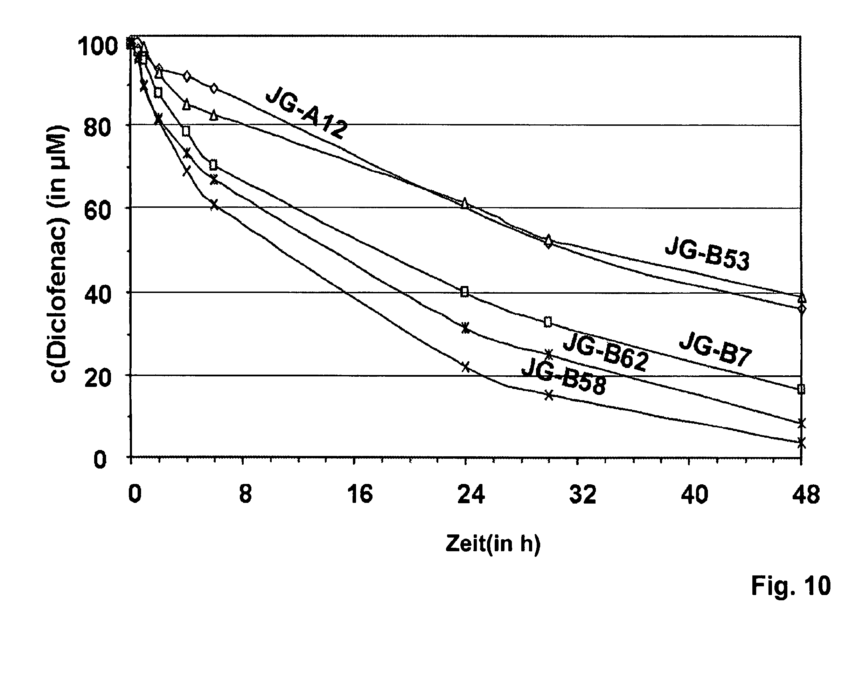

**Figur 10** vergleicht den Diclofenac-Abbau durch 3 mg ZnO-Nanopartikel hergestellt mit 10 mg S-Layer verschiedener Stämme.

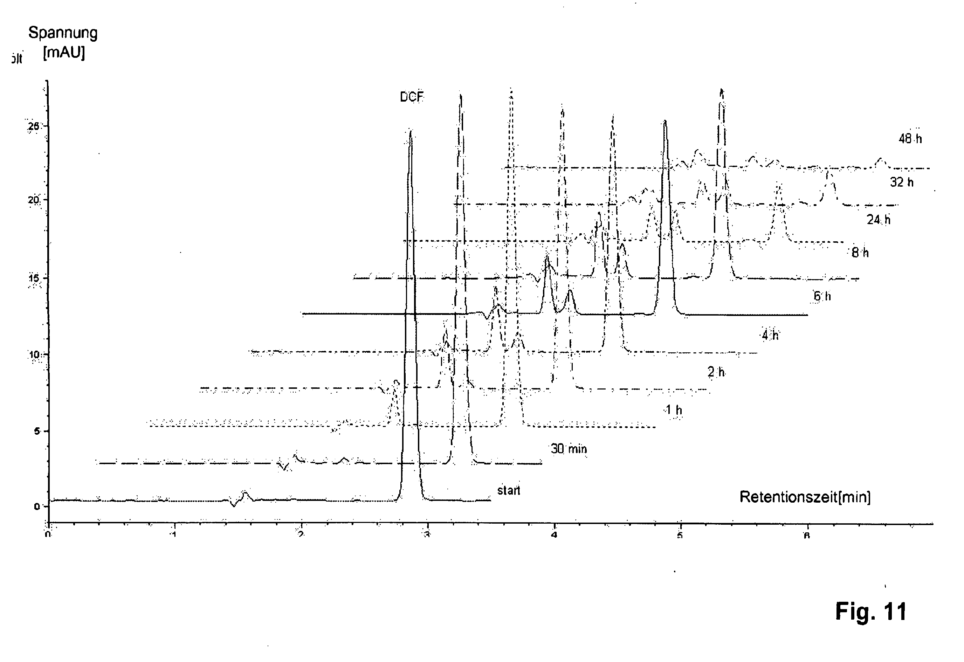

**Figur 11** zeigt ein HPLC Chromatogramm der fotokatalytischen Eliminierung von 100 µM Diclofenac (DCF) mittels biomolekular auf 1,6 mg S-Layern (*Lysinibacillus sphaericus* JG-A12) erzeugten ZnO-Nanopartikel bei Bestrahlung mit Licht der Wellenlänge 365 nm.

## Patentansprüche

1. Nanostruktur, enthaltend
a) S-Layer-Proteine, an die Nukleinsäure-Aptamere und/oder Antikörper, die spezifische Bindungseigenschaften für ein Zielmolekül aufweisen, gebunden sind und
b) an die S-Layer- Proteine gebundene anorganische Nanopartikel und/oder anorganische bzw. organische Fluoreszenzfarbstoffe oder weitere Biomoleküle, die als funktionelle Bestandteile unterschiedliche Funktionen erfüllen,
wobei die S-Layer-Proteine eine kristalline Schicht auf einer Oberfläche eines Substrats ausbilden oder in einer geeigneten Flüssigkeit als Suspension vorliegen,
wobei die Nukleinsäure-Aptamere und/oder Antikörper so geordnet an die S-Layer-Proteine gebunden sind, dass sie die gebundenen Zielmoleküle in die Nähe der funktionellen Bestandteile bringen, die in spezifische Wechselwirkung mit dem Zielmolekül treten.

2. Struktur nach Anspruch 1, **dadurch gekennzeichnet, dass** die Aptamere und/oder Antikörper spezifisch Schwermetalle, Arzneimittelsubstanzen oder organische Moleküle in wässrigen Systemen binden.

3. Struktur nach Anspruch 1 oder 2, **dadurch gekennzeichnet, dass** S-Layer-Proteine der Bakteriengattungen ausgewählt aus *Bacillus, Lactobacillus, Lysinibacillus, Geobacillus, Deinococcus, Sporosarcina* verwendet werden.

4. Struktur nach einem der Ansprüche 1 bis 3, **dadurch gekennzeichnet, dass** die Nanopartikel aus Magnetit sind oder katalytische Eigenschaften, vorzugsweise photokatalytische Eigenschaften, aufweisen.

5. Struktur nach einem der Ansprüche 1 bis 4, **dadurch gekennzeichnet, dass** die Struktur zwei zueinander unterschiedliche Moleküle enthält, die zur Erzeugung eines Förster-Resonanz-Energie-Transfers (FRET) geeignet sind, wobei dazu an die Struktur bevorzugt zwei zueinander unterschiedliche organische und/oder anorganische Fluoreszenzfarbstoffe gebunden sind.

6. Struktur nach Anspruch 5 **dadurch gekennzeichnet, dass** mindestens einer der beiden Fluoreszenzfarbstoffe ein Quantenpunkt (quantum dot) ist.

7. Sensor, enthaltend S-Layer-Proteine und zwei zueinander unterschiedliche Moleküle, die zur Erzeugung eines Förster-Resonanz-Energie-Transfer (FRET) geeignet sind, wobei die S-Layer-Proteine eine kristalline Schicht auf einer Oberfläche eines Substrats ausbilden oder in einer geeigneten Flüssigkeit als Suspension vorliegen, wobei an die selbstorganisierenden Proteine Aptamere und/oder Antikörper gebunden sind, wobei durch die Bindung an die Aptamere oder Antikörper die nachzuweisenden Moleküle und/oder Atome in die Nähe des Donor/Akzeptorpaares gebracht werden, wodurch das FRET-Signal modifiziert und die Änderung messtechnisch erfasst wird.

8. Sensor nach Anspruch 7, **dadurch gekennzeichnet, dass** die Aptamere und/oder Antikörper spezifisch Schwermetalle oder organische Moleküle oder Arzneimittelsubstanzen in wässrigen Systemen binden.

9. Sensor nach Anspruch 7 oder 8, **dadurch gekennzeichnet, dass** mindestens einer der beiden Fluoreszenzfarbstoffe ein Quantenpunkt (quantum dot) ist.

10. Sensor nach einem der Ansprüche 7 bis 9, **dadurch gekennzeichnet, dass** S-Layer-Proteine der Bakteriengattungen ausgewählt aus *Bacillus, Lactobacillus, Lysinibacillus, Geobacillus, Deinococcus, Sporosarcina* verwendet werden.

11. Verwendung der Struktur nach einem der Ansprüche 1 bis 6 oder des Sensors nach einem der Ansprüche 7 bis 10 in der Biotechnologie, der Verfahrenstechnik, der Umwelttechnik, der Pharmazie und/oder der biomedizinischen Analytik.

12. Verwendung der Struktur nach einem der Ansprüche 1 bis 6 zur Beseitigung von Schadstoffen und/oder Pathogenen, für die DNA-Analytik, für die Herstellung von Filtermaterialien, für Separationsverfahren und/oder Katalysatoren.

## Claims

1. Nanostructure, comprising
a) S-layer proteins to which nucleic acid aptamers and/or antibodies that exhibit specific binding properties for a target molecule are bound; and
b) inorganic nanoparticles and/or inorganic or organic fluorescent dyes or other biomolecules that act as functional components fulfilling various functions and that are bound to the S-layer proteins,
wherein the S-layer proteins form a crystalline layer on a surface of a substrate or are present in a suitable liquid as a suspension,
wherein the nucleic acid aptamers and/or antibodies are attached to the S-layer proteins in an ordered manner such that they bring the bound target molecules into proximity to the functional components that specifically interact with the target molecule.

2. Structure according to claim 1 **characterized by that** the aptamers and/or antibodies specifically bind heavy metals, drug substances or organic molecules in aqueous systems.

3. Structure according to claim 1 or 2 **characterized by that** S-layer proteins of the bacterial genera selected from *Bacillus, Lactobacillus, Lysinibacillus, Geobacillus, Deinococcus, Sporosarcina* are used.

4. Structure according to any of the claims 1 to 3 **characterized by that** the nanoparticles are of magnetite or possess catalytic properties, preferably photocatalytic properties.

5. Structure according to any of the claims 1 to 4 **characterized by that** the structure contains two molecules that are different to each other, suitable for generating a Förster resonance energy transfer (FRET), wherein for that purpose preferably two organic and/or inorganic fluorescent dyes that are different to each other are attached to the structure.

6. Structure according to claim 5 **characterized by that** at least one of both fluorescent dyes is a quantum dot.

7. Sensor, comprising S-layer proteins and two molecules that are different to each other suitable for generating a Förster resonance energy transfer (FRET), wherein the S-layer proteins form a crystalline layer on a surface of a substrate or are present in a suitable liquid as a suspension, wherein aptamers and/or antibodies are bound to the self-organizing proteins, wherein due to binding to the aptamers or antibodies the molecules and/or atoms to be detected are brought into proximity to the donor/acceptor pair so that the FRET-signal is modified and the modification is recorded by measurement.

8. Sensor according to claim 7 **characterized by that** the aptamers and/or antibodies specifically bind heavy metals or organic molecules or drug substances in aqueous systems.

9. Sensor according to claim 7 or 8 **characterized by that** at least one of both fluorescent dyes is a quantum dot.

10. Sensor according to any of the claims 7 to 9 **characterized by that** S-layer proteins of the bacterial genera selected from *Bacillus, Lactobacillus, Lysinibacillus, Geobacillus, Deinococcus, Sporosarcina* are used.

11. Use of the structure according to any of the claims 1 to 6 or of the sensor according to any of the claims 7 to 10 in biotechnology, process engineering, environmental technology, pharmaceutics and/or biomedical analytics.

12. Use of the structure according to any of the claims 1 to 6 for the disposal of contaminants and/or pathogens, for DNA-analytics, for the production of filter materials, for separation processes and/or catalysts.

## Revendications

1. Nanostructure contenant
a) des protéines de couche S auxquelles sont liés des aptamères d'acide nucléique et/ou des anticorps qui présentent des propriétés spécifiques de liaison à une molécule cible et
b) des nanoparticules inorganiques et/ou des colorants de fluorescence inorganiques ou organiques ou d'autres biomolécules qui remplissent différentes fonctions en tant que composants fonctionnels, liés aux protéines de couche S,
où les protéines de couche S forment une couche cristalline sur une surface d'un substrat ou se présentent dans un fluide approprié sous la forme d'une suspension, où les aptamères d'acide nucléique et/ou les anticorps sont liés aux protéines de couche S en étant disposés de façon telle qu'ils portent les molécules cibles liées à la proximité des composants fonctionnels, qui entrent en interaction spécifique avec la molécule cible.

2. Structure selon la revendication 1, **caractérisée en ce que** les aptamères et/ou les anticorps lient de façon spécifique des métaux lourds, des substances médicamenteuses ou des molécules organiques dans des systèmes aqueux.

3. Structure selon la revendication 1 ou 2, **caractérisée en ce que** l'on utilise des protéines de couche S des espèces bactériennes sélectionnées parmi *Bacillus, Lactobacillus, Lysinibacillus, Geobacillus, Deinococcus, Sporosarcina.*

4. Structure selon l'une des revendications 1 à 3, **caractérisée en ce que** les nanoparticules sont en magnétite ou présentent des propriétés catalytiques, de préférence des propriétés photocatalytiques.

5. Structure selon l'une des revendications 1 à 4, **caractérisée en ce que** la structure contient deux molécules différentes l'une de l'autre qui sont appropriées pour générer un transfert d'énergie de résonance de Förster (FRET), ou de préférence étant pour ce faire deux colorants de fluorescence organiques et/ou inorganiques différents l'un de l'autre sont liés à la structure.

6. Structure selon la revendication 5, **caractérisée en ce qu'**au moins un des deux colorants de fluorescence est un point quantique (quantum dot).

7. Capteur contenant des protéines de couche S et deux molécules différentes l'une de l'autre qui sont appropriées pour générer un transfert d'énergie de résonance de Förster (FRET), où les protéines de couche S forment une couche cristalline sur une surface d'un substrat ou se présentent en tant que suspension dans un fluide approprié, où des aptamères et/ou des anticorps sont liés aux protéines à auto-organisation, et où, par la liaison aux aptamères ou aux anticorps, les molécules et/ou atomes à détecter sont amenés à la proximité de la paire donneur/accepteur, suite à quoi le signal de FRET est modifié et la modification est enregistrée par une technique de mesure.

8. Capteur selon la revendication 7, **caractérisé en ce que** les aptamères et/ou les anticorps lient de façon spécifique des métaux lourds ou des molécules organiques ou des substances médicamenteuses dans des systèmes aqueux.

9. Capteur selon la revendication 7 ou 8, **caractérisé en ce qu'**au moins un des deux colorants de fluorescence est un point quantique (quantum dot).

10. Capteur selon l'une des revendications 7 à 9, **caractérisé en ce que** l'on utilise des protéines de couche S des espèces bactériennes sélectionnées parmi *Bacillus, Lactobacillus, Lysinibacillus, Geobacillus, Deinococcus, Sporosarcina.*

11. Utilisation de la structure selon l'une des revendications 1 à 6 ou du capteur selon l'une des revendications 7 à 10 en biotechnologie, en technique de procédés, en génie écologique, en pharmacie et/ou en analyse biomédicale.

12. Utilisation de la structure selon l'une des revendications 1 à 6 afin d'éliminer des polluants et/ou des agents pathogènes, pour l'analyse d'ADN, pour la fabrication de matériaux de filtre, pour des procédés de séparation et/ou des catalyseurs.


### SEQUENCE LISTING

<110> Forschungszentrum Dresden - Rossendorf e. V.
<120> Hochgeordnete Nanostruktur und Sensor und deren Verwendung
<130> 00466P0001DEEP
<150> DE 10 2008 014 298
   <151> 2008-03-10
<160> 13
<170> PatentIn version 3.3
<210> 1
   <211> 27
   <212> DNA
   <213> Artificial
<220>
   <223> Aptamer
<400> 1
   acctggggga gtattgcgga ggaaggt 27
<210> 2
   <211> 60
   <212> DNA
   <213> Artificial
<220>
   <223> Aptamer
<400> 2
   tgaggcgggt gggtgggttg aatatgctga ttaccccatc ggagaacgtt aaggcgcttc 60
<210> 3
   <211> 40
   <212> DNA
   <213> Artifcial
<400> 3
   cgaccgcagg tgcactgggc gacgtctctg ggtgtggtgt 40
<210> 4
   <211> 40
   <212> DNA
   <213> Artificial
<220>
   <223> Aptamer
<400> 4
   cgacgcgcgt tggtggtgga tggtgtgtta cacgtgttgt 40
<210> 5
   <211> 40
   <212> DNA
   <213> Artificial
<220>
   <223> Aptamer
<400> 5
   acgttgacgc tggtgcccgg ttgtggtgcg agtgttgtgt 40
<210> 6
   <211> 40
   <212> DNA
   <213> Artificial
<220>
   <223> Aptamer
<400> 6
   cgagttgagc cgggcgcggt acgggtactg gtatgtgtgg 40
<210> 7
   <211> 40
   <212> DNA
   <213> Artificial
<220>
   <223> Aptamer
<400> 7
   gggcagcggt ggtgtggcgg gatctggggt tgtgcggtgt 40
<210> 8
   <211> 40
   <212> DNA
   <213> Artificial
<220>
   <223> Aptamer
<400> 8
   ggaggaacgg gttccagtgt ggggtctatc ggggcgtgcg 40
<210> 9
   <211> 40
   <212> DNA
   <213> Artificial
<220>
   <223> Aptamer
<400> 9
   cgggagggcg gggtgtggta tgtattgagc gtggtccgtg 40
<210> 10
   <211> 40
   <212> DNA
   <213> Artificial
<220>
   <223> Aptamer
<400> 10
   cccccggcag gccacggctt gggttggtcc cactgcgcgt 40
<210> 11
   <211> 40
   <212> DNA
   <213> Artificial
<220>
   <223> Aptamer
<400> 11
   gtgcgcacgc taggtggtga tgctgtgcta cacgtgttgt 40
<210> 12
   <211> 40
   <212> DNA
   <213> Artificial
<220>
   <223> Aptamer
<400> 12
   gggggcacac atgtaggtgc tgtccaggtg tggttgtggt 40
<210> 13
   <211> 40
   <212> DNA
   <213> Artifcial
<400> 13
   gggcgggggt gctgggggaa tggagtgctg cgtgctgcgg 40